# EUROPEAN PATENT SPECIFICATION

(11) **EP 2 632 613 B1**
(45) Date of publication and mention of the grant of the patent: **30.08.2017**
(21) Application number: 11782325.2
(22) Date of filing: 28.10.2011
(51) Int. Cl.: B08B 17/06, B82Y 30/00

(54) **ENGINEERED SURFACES FOR REDUCING BACTERIAL ADHESION**
MANIPULIERTE OBERFLÄCHEN ZUR REDUKTION DER BAKTERIELLEN ADHÄSION
SURFACES MODIFIÉES POUR LA RÉDUCTION DE L'ADHÉSION BACTÉRIENNE

(30) Priority: 27.06.2011 US 201161501541 P; 28.10.2010 US 407820 P; 28.10.2010 US 407813 P; 28.10.2010 US 407806 P
(43) Date of publication of application: 04.09.2013
(73) Proprietor: 3M Innovative Properties Company, St. Paul, MN 55133-3427 (US)
(72) Inventor: BOMMARITO, G. Marco, Saint Paul, Minnesota 55133-3427 (US); SCHOLZ, Matthew T., Saint Paul, Minnesota 55133-3427 (US); SVAROVSKY, Michael J., Saint Paul, Minnesota 55133-3427 (US); YARWOOD, Jeremy M., Saint Paul, Minnesota 55133-3427 (US); SCHNOBRICH, Scott M., Saint Paul, Minnesota 55133-3427 (US); DEVOE, Robert J., Saint Paul, Minnesota 55133-3427 (US); ZHANG, Jun-Ying, Saint Paul, Minnesota 55133-3427 (US); SMITH, Terry L., Saint Paul, Minnesota 55133-3427 (US)
(74) Representative: Vossius & Partner Patentanwälte Rechtsanwälte mbB
(86) International application number: PCT/US2011/058409
(87) International publication number: WO 2012/058605

(56) References cited:
- WO-A2-2009/002644
- DE-A1- 19 818 956
- DE-A1- 19 950 452
- US-A1- 2006 024 508
- US-A1- 2007 231 542
- US-A1- 2010 226 943
- XUE-MEI LI ET AL: "What do we need for a superhydrophobic surface? A review on the recent progress in the preparation of superhydrophobic surfaces", WHAT DO WE NEED FOR A SUPERHYDROPHOBIC SURFACE? A REVIEW ON THE RECENT PROGRESS IN THE PREPARATION OF SUPERHYDROPHOBIC SURFACES,, vol. 36, 31 January 2007 (2007-01-31), pages 1350-1368, XP002667653,

## Description

### Background

Biofilms are structured communities of microorganisms encased in an extracellular polymeric matrix that typically are tenaciously adhered to the surface of biomaterials and host tissue. Bacterial biofilms are a significant issue in the development of materials that are exposed to aqueous and body fluids for prolonged periods for several different application areas: medical devices, filtration systems for food processing and other industrial applications, coatings for marine structures and other anti-fouling applications. Bacteria living in a biofilm are considerably more resistant to host defenses and antibiotic or antimicrobial treatments, when compared to "free" pathogens, and thereby increase the potential for infections during the use of in-dwelling and other tissue contacting devices.

Biofilms are believed to have a significant role in catheter associated urinary tract infections (CAUTI), catheter associated bloodstream infections, and ventilator associated pneumonia (VAP). CAUTIs comprise the largest percentage of hospital acquired infections (HAIs) and are the second most common cause of nosocomial bloodstream infections. VAP has the highest morbidity of all HAIs, as roughly 15% of patients with VAP will die. VAP may also be the most expensive HAI to treat ($20,000-$50,000 per episode), and has an incident rate between 25% and 40%.

By way of example, and without wishing to be bound by theory, biofilm formation on urinary catheter surfaces may proceed as follows: 1) The catheter surface is initially colonized by bacteria (some of them urease-producing bacteria) originally present on the periurethral skin and able to migrate into the bladder between the epithelial surface of the urethra and the catheter once the catheter is inserted. The adsorption of these cells to the catheter surface may be facilitated by the formation of an organic conditioning film made up largely of adsorbed proteins. 2) A bacterial biofilm community forms, encased primarily by a matrix of bacterial exopolysaccharide. The pioneer biofilm forming bacteria that initially cause urinary tract infections (UTIs) are typically *S. epidermidis, E. coli* or *E. faecalis,* with *E. coli* the overwhelming cause of CAUTI. At longer catheterization times, other species appear including *P. aeruginosa, P. mirabillis,* and *K*. *pneumoniae.* These latter stage bacteria are more difficult to treat with antibiotics while the catheter is in place. 3) The presence of a growing biofilm, including bacterial species that are capable of producing urease, leads to an elevation of the urine's pH due to the action of urease on urea. 4) As the urine becomes alkaline, calcium phosphate and magnesium ammonium phosphate crystals precipitate and accumulate in the biofilm matrix growing on the catheter surface. 5) Continued crystal formation in the alkaline urine and continued growth of the biofilm lead to severe encrustation and eventually blockage of the device which necessitates re-catherization of the patient. Thus, preventing colonization and biofilm formation on the catheter could play a large role preventing CAUTIs.

Attempts have been made to provide surfaces that are inherently antimicrobial, either by composition or use of antimicrobial drug delivery systems. These surfaces can be insufficiently effective in reducing biofilm formation for three important reasons: 1) when used as a delivery system, antimicrobial or active agents may be exhausted well before the end of the service lifetime of the medical article; 2) the surface antimicrobial properties are eventually impaired as dead cells, the high organic load in the urethra, and other adsorbed biomaterial mask the antimicrobial properties of that surface; and 3) antimicrobial agents in the catheter material or in an external coating fail to elute sufficiently.

Further attempts have been made to provide surfaces having an optimized engineered roughness index. Such attempts suggest that increased surface complexity is needed to sufficiently disrupt microorganism adhesion, indicating that simpler patterns are inadequate. While effective at reducing microbial adhesion in certain circumstances, these surfaces may be costly to reproduce on a sufficient scale. Effectively controlling and preventing biofilm formation long-term requires the creation of biomaterial surfaces that retain superior anti-adhesive properties throughout the useful life of the base medical article.

DE 199 50 452 A1 discloses an article having a structured surface which is capable of inhibiting proliferation of cells. The surface has protrusions with an average height of from 50 nm to 10 µm and an average separation of from 50 nm to 10 µm.

WO 2009/002644 A2 discloses a method of making a hierarchical article. The method comprises providing a substrate that comprises a nano featured pattern; adding a layer to the substrate; and generating a microstructured pattern in the layer.

### Summary

An apparatus having bacterial anti-adhesion properties and a method of creating an anti-adhesion surface as recited in the independent claims are provided. The dependent claims define embodiments.

A medical article of the present disclosure includes a surface topography (i.e., the surface features of an object or region thereof) for resisting bioadhesion of microorganisms. The surface topography may be integral with or affixed to an exterior and/or interior surface of the article. The engineered surface has a topography comprising at least one pattern or arrangement, which can typically be defined by a plurality of unit cells. Each unit cell comprises at least one engineered structure protruding from or projected into that surface. The engineered structure may be a microstructure or a nanostructure. Each engineered structure can have further directed nanofeatures, typically possessing smaller dimensions, protruding therefrom. In certain embodiments, the unit cell is at least partially defined by a dimension at least approximating the pitch (i.e., distance between adjacent structures as measured centroid to centroid) between adjacent engineered structures. In some embodiments of the present disclosure, the entire engineered surface is comprised of a single repeating unit cell geometry. In further embodiments, the plurality of unit cells are tiled and/or tessellated, in that the outer boundaries of a particular unit cell are directly adjacent the outer boundaries of any neighboring unit cell.

In certain embodiments, the engineered structures includes a base having at least one dimension of at least one cross section no less than 0.5 microns and no greater than 50 microns. The pitch between adjacent eningeered structures is typically at least the smallest dimension of the structure and may be no greater than 5 times said smallest dimension.

Surface topographies according to the disclosure resist bioadhesion as compared to a surface without such topography. Surface topographies according to the invention can be created by affixing a film or other substrate containing the plurality of microstructures and nanostructures to a target surface of the medical article or by microreplicating the structural features directly to the surface of the article. When microreplicated, the resulting structures will be monolithically integrated with the underlying article. In other embodiments, the engineered structures can be created by photolithography.

The engineered surfaces of the present invention can reduce the colonization of target microorganisms by at least 50% over 14 days compared to the colonization on a flat surface comprised of the same material, in the absence of antimicrobials and/or with any antimicrobial agents inactivated, according to the assay as set out in the Examples below. In preferred embodiments, the reduction in colonization can be at least 75%. In even more preferred embodiments, the reduction in colonization can be as high as 90%. In certain embodiments, the target organisms comprise *Pseudomonas aeruginosa, Staphylococcus aureus*; and methicillin-resistant *Staphylococcus aureus.*

The engineered surfaces containing both engineered structures and directed nanofeatures can reduce the colonization of target microorganisms over 14 days compared to the colonization on a surface including microstructures of the same geometry and comprised of the same material, in the absence of antimicrobials and/or with any antimicrobial agents inactivated, according to the assay as set out in the Examples below. Furthermore, the engineered surfaces containing both engineered structures and nanofeatures can reduce colonization compared to surfaces including only nanofeatures of the same geometry and material.

Surfaces modified according to the present disclosure are equally or at least comparatively effective at resisting bioadhesion of target organisms in comparison to more complex topographies. The present inventors have found that both structure size and spacing impact the surface resistance to bioadhesion, particularly when both are commensurate with the size of the target microorganism. This discovery allows for the creation of repeating patterns of similarly sized microstructures in simplified arrays. These simplified arrays are expected to significantly improve the process efficiency by reducing shrinkage, providing more predictable and more uniform shrinkage, as well as providing more thermal uniformity in thermoplastic and thermoset molds. Thus, the preferred topographies of the present invention may be easily replicated with enhanced feature fidelity and manufactured on an industrial scale at appreciably reduced cost. The expected improvement in production cost and quality makes the engineered surfaces of the present invention particularly suited for a wide variety of potential applications.

As used herein "geometry" refers to the size and shape of a feature.

As used herein "base" of a structure is defined at the plane of the substrate surface from which the microstructure emerges. In "negative" microstructures which project into a substrate (e.g. holes) the base is defined at the plane of the substrate surface, i.e. at the entrance to the "hole".

As used herein, a "microstructure" is a structure or feature having a recognizable geometric shape defined by a volume that projects out the base plane of a surface or an indented volume which projects into the surface. Such structures include at least one microscale dimension and typically include a base having cross sectional dimensions no less than 0.5 microns and no greater than 5 microns.

As used herein, a "continuous structure" is a micro or nanostructure having a length at least 1 mm. In certain implementations, a continuous structure extends along an entire dimension of the engineered surface. For example and as further described below, a continuous structure can be segmented along the x-axis and/or the z-axis, but not the y-axis.

As used herein, an "engineered structure" shall mean a structure deliberately formed into and integral with a surface. An engineered structure may be created, for example, by microreplicating a specific pattern unto a surface. An engineered microstructure is distinct from structures produced by random application of particles, by spraying, adhesive bonding, etc, to a surface and can include microstructures and nanostructures.

As used herein, the term "microstructured surface" is generally used to refer to a surface that comprises microstructures or microstructured features.

The term "microreplicate" and derivatives thereof, is generally used to refer to the production of a microstructured surface through a process where the structured surface features retain an individual feature fidelity during and after manufacture.

The terms "nanostructure" and "nanofeature" are used interchangeably and describe structures having a least one nanoscale dimension. In certain embodiments, the nanofeatures include no dimension exceeding 1 micron. As used herein, the term "directed nanofeature" and variations thereof means a nanofeature deliberately created on a surface. Exemplary methods for creating directed nanofeatures include etching (e.g., reactive-ion etching) and deposition. Certain exemplary directed nanofeatures include dentritic projections having a height between 25 nm and 350 nm (preferably between 100 nm and 250 nm) and a diameter (or width) of 30 to 80 nm.

As used herein, the term "orifice device" means a medical device intended to be inserted into a natural orifice of a human and includes, but is not limited to, urinary catether, vascular access catethers, and endotracheal tubes.

As used herein, the term "pitch" identifies the distance between the centroids of adjacent microstructures. The pitch is measured from the centroid of a microstructure (i.e., the geometric center) to the centroid of an adjacent microstructure.

As used herein, the terms "height" , "base" and "top" are for illustrative purposes only, and do not necessarily define the orientation or the relationship between the surface and the microstructure. For example, the "height" of a microstructure projected into a surface can be considered the same as the depth of recess created, and the "top" the bottom said recess. Accordingly, the terms "height" and "depth", and "top" and "bottom" should be considered interchangeable.

The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As recited herein, all numbers should be considered modified by the term "about".

As used herein, "a," "an," "the," "at least one," and "one or more" are used interchangeably. Thus, for example, an article comprising an "engineered surface" can be interpreted to comprise one or more "engineered surfaces"

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### Brief Description of the Drawings

The invention will be further described with reference to the drawings, wherein corresponding reference characters indicate corresponding parts throughout the several views, and wherein:
Fig. 1 is a cross-sectional view of a medical article including an engineered surface according to embodiments of the disclosure comprising both engineered structures and directed nanofeatures.
Fig. 2 illustrates a surface comprising a series of discrete recesses according to an embodiment of the disclosure.
Figs. 3a-c illustrate various shapes of microstructures according to the present description.
Fig. 4 is a perspective view of an engineered surface according to an embodiment of the disclosure.
Figs. 5a-b illustrate cross-sectional views of a nanocavity and a nanopost according to certain implementations of the disclosure
Figs. 6a-e illustrate a variety of engineered surfaces according to certain embodiments of the disclosure.
Figs. 7a-f illustrate configurations of repeating unit cells according to certain embodiments of the disclosure.
Figs. 8a-c illustrate configurations of repeating unit cells according to certain embodiments of the disclosure.
Figs. 9a-b illustrate a two-level engineered surface according to certain embodiments of the disclosure.
Fig. 10 illustrates a two-level engineered surface according to certain embodiments of the disclosure.
Figs. 11a-d illustrate a two-level engineered surface according to certain embodiments of the disclosure.

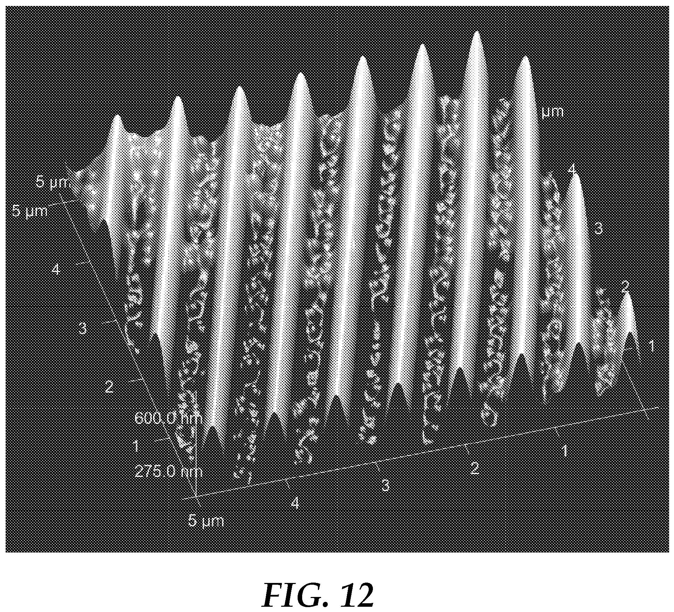

Fig. 12 illustrates a two-level engineered surface according to another embodiment of the disclosure.

While the above-identified figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope of the principles of the invention as defined by the claims.

### Detailed Description

Medical articles of the present disclosure provide reduced colonization of target microorganisms, and thus biofilm formation, on surfaces of the article that include a plurality of engineered structures having a plurality of directed nanofeatures disposed thereon. The engineered structures may comprise either microstructures or nanostructures. In certain embodiments, the nanofeatures are disposed in areas of the surface not occupied by engineered structures (i.e., in between engineered structures). In certain embodiments, the engineered surface has at least one unit cell defined at least partially by the pitch between adjacent engineered structures protruding from or projected into that surface. Each unit cell can be defined in a single plane and includes at least one structure having a single geometry and orientation. The unit cells can be arranged to be tiled or at least substantially tessellated, such that the boundary region of any one unit cell is directly adjacent to the boundary region of any neighboring unit cell (i.e., there is no deliberate space between the unit cells).

In some embodiments of the present disclosure, at least a portion of the engineered surface is comprised of a single repeating unit cell, and accordingly one engineered structure geometry. In other embodiments, at least a portion of the surface comprises a plurality of unit cells that include a plurality of structure geometries.

The term "microorganism" is generally used to refer to any prokaryotic or eukaryotic microscopic organism, including without limitation, one or more of bacteria (e.g., motile or vegetative, Gram positive or Gram negative), bacterial spores or endospores, algae, fungi (e.g., yeast, filamentous fungi, fungal spores), mycoplasmas, and protozoa, as well as combinations thereof. In some cases, the microorganisms of particular interest are those that are pathogenic, and the term "pathogen" is used to refer to any pathogenic microorganism. Examples of pathogens can include, but are not limited to, both Gram positive and Gram negative bacteria, fungi, and viruses including members of the family *Enterobacteriaceae,* or members of the family *Micrococaceae,* or the genera *Staphylococcus* spp., *Streptococcus,* spp., *Pseudomonas* spp., *Enterococcus* spp., *Salmonella* spp., *Legionella* spp., *Shigella* spp., *Yersinia* spp., *Enterobacter* spp., *Escherichia* spp., *Bacillus* spp., *Listeria* spp., *Campylobacter* spp., *Acinetobacter* spp., *Vibrio* spp., *Clostridium* spp., *Klebsiella* spp., *Proteus* spp. and *Corynebacterium* spp. Particular examples of pathogens can include, but are not limited to, *Escherichia coli* including enterohemorrhagic *E. coli* e.g., serotype O157:H7, O129:H11 *Pseudomonas aeruginosa*; *Bacillus cereus*; *Bacillus anthracis*; *Salmonella enteritidis*; *Salmonella enterica* serotype Typhimurium; *Wisteria monocytogenes; Clostridium botulinum; Clostridium perfringens; Staphylococcus aureus;* methicillin-resistant *Staphylococcus aureus*; *Campylobacter jejuni; Yersinia enterocolitica; Vibrio vulnificus; Clostridium difficile*; vancomycin-resistant *Enterococcus; Klebsiella pnuemoniae; Proteus mirabilus* and *Enterobacter [Cronobacter] sakazakii.*

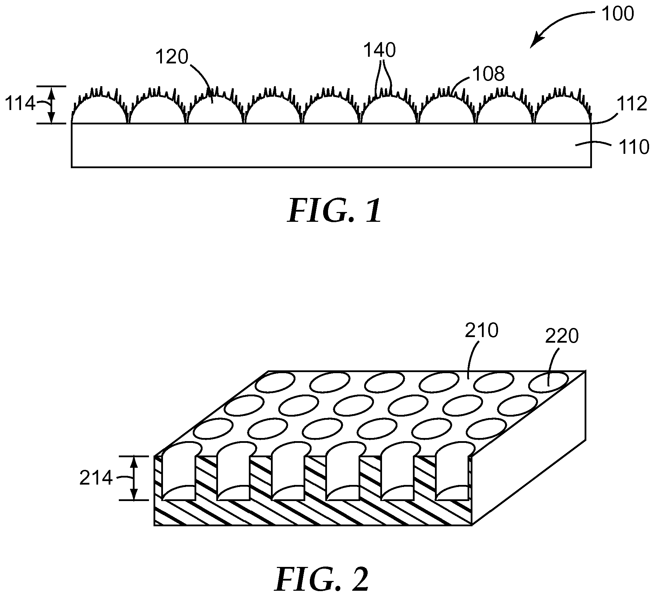

A medical article 100 including at least one engineered surface 110 for contact with a tissue or fluid is depicted in Figure 1a. In some embodiments, the engineered surface defines a portion of the exterior surface of medical article 100. In other embodiments, the engineered surface 110 defines a portion of the interior surface of a medical article 100. In yet other embodiments, the engineered surface may comprise at least a portion of both the interior and exterior surfaces of the medical article 100. Suitable medical articles for use with the invention include, but are not limited to: nasal gastric tubes, wound contact layers, blood stream catheters, dialysis catheters and tubing stents, pacemaker shells, heart valves, orthopedic implants such as hips, knees, shoulders, etc., periodontal implants, orthodontic brackets and other orthodontic appliances, dentures, dental crowns, contact lenses, intraocular lenses, soft tissue implants (breast implants, penile implants, facial and hand implants, etc..), surgical tools, sutures including degradable sutures, cochlear implants, tympanoplasty tubes, shunts including shunts for hydrocephalus, post surgical drain tubes and drain devices, urinary catheters, endotraecheal tubes, heart valves, wound dressings, other implantable devices, and other indwelling devices.

The medical article 100 includes a plurality of engineered structures 120 molded or integral with at least a portion of the engineered surface 110. In Figure 1, the engineered structures 120 are depicted as dome-shaped features projecting or protruding from the engineered surface 110. In other embodiments as depicted in Figure 2, it may be preferred that the plurality engineered structures 220 are projected into the engineered surface 210 at height (i.e., depth) 214, creating a series of disconnected or discrete recesses. In such embodiments the projected or "negative" structures may improve the anti-adhesion capabilities of the engineered surfaces in comparison to a projected or "positive" equivalent structure.

Without wishing to be bound by theory, the improved reduction in microorganism adhesion for negative engineered structures could be due to a difference in how negative and positive structures are wetted by fluid (growth media) covering those surfaces. Whereas a positive structure is defined by an interconnected or continuous channel structure, the equivalent negative pattern is defined by a disconnected or discrete recess or channel structure (e.g., the pockets may form discontinuous channels). A somewhat high surface tension liquid contacting a positive structure may be able to fully wet that surface because the entrapped air may be displaced more readily due to the presence of an interconnected channel structure. The same liquid on a negative structure, with discrete micron sized recesses, may not be able to fully wet that surface because the surface tension over an opening of that size is difficult to break, and the air or other gasses entrapped in those recesses cannot be easily displaced. The net result is a lower fractional area available for bacterial contact and subsequent adhesion.

Formed on at least one of the engineered structures 120 are a plurality of directed nanofeatures 140. Generally, the engineered structures and nanofeatures may be composed of all or substantially all of the same material. More specifically, the engineered structures and nanofeatures may be made of a curable, thermoset material. In some embodiments, that material is a majority silicone polymer by weight. In at least some embodiments, the silicone polymer will be polydialkoxysiloxane such as poly(dimethylsiloxane) (PDMS), such that the microstructures are made of a material that is a majority PDMS by weight. More specifically, the microstructures may be all or substantially all PDMS. For example, the microstructures may each be over 95wt.% PDMS. In certain embodiments the PDMS is a cured thermoset composition formed by the hydrosilylation of silicone hydride (Si-H) functional PDMS with unsaturated functional PDMS such as vinyl functional PDMS. The Si-H and unsaturated groups may be terminal, pendant, or both. In other embodiments the PDMS can be moisture curable such as alkoxysilane terminated PDMS.

In some embodiments, other silicone polymers besides PDMS may be useful, for example, silicones in which some of the silicon atoms have other groups that may be aryl, for example phenyl, alkyl, for example ethyl, propyl, butyl or octyl, , fluororalkyl, for example 3,3,3-trifluoropropyl, or arylalkyl, for example 2-phenylpropyl. The silicone polymers may also contain reactive groups, such as vinyl, silicon-hydride (Si-H), silanol (Si-OH), acrylate, methacrylate, epoxy, isocyanate, anhydride, mercapto and chloroalkyl. These silicones may be thermoplastic or they may be cured, for example, by condensation cure, addition cure of vinyl and Si-H groups, or by free-radical cure of pendant acrylate groups. They may also be cross-linked with the use of peroxides. Such curing may be accomplished with the addition of heat or actinic radiation. Other useful polymers may be thermoplastic or thermoset and include polyurethanes, polyolefins including metallocene polyolefins, polyesters such as elastomeric polyesters (e.g. Hytrel), biodegradable polyesters such as polylactic, polylactic/glycolic acids, copolymers of succinic acid and diols, and the like, fluoropolymers including fluoroelastomers, polyacrylates and polymethacrylates. Polyurethanes may be linear and thermoplastic or thermoset. Polyurethanes may be formed from aromatic or aliphatic isocyanates combined with polyester or polyether polyols or a combination thereof. In another embodiment, polymers with a glass transition temperature of less than 25°C are useful. Particularly useful are polymers with a glass transition temperature of less than about 10°C. In at least some embodiments, the microstructures may be an elastomer. An elastomer may be understood as a polymer with the property of viscoelasticity (or elasticity) generally having suitably low Young's modulus and high yield strain compared with other materials. The term is often used interchangeably with the term rubber, although the latter is preferred when referring to cross-linked polymers.

Polymers may also be filled with suitable organic or inorganic fillers and for certain applications the fillers are radioopaque. The polymers may contain other additives such as antimicrobial agents (including antiseptics and antibiotics), dyes, mold release agents, antioxidants, plasticizers, and the like. Suitable antimicrobials can be incorporated into or deposited onto the polymers. Suitable preferred antimicrobials include those described in US Publication. Nos. 2005/0089539 and 2006/0051384 to Scholz et al. and US Publication Nos. 2006/0052452 and 2006/0051385 to Scholz. The engineered surfaces of the present invention also may be coated with antimicrobial coatings such as those disclosed in International Application No. PCT/US2011/37966 to Ali et al.

In other embodiments, the engineered structures are composed of a different material than the directed nanofeatures. In some embodiments, the nanofeatures may comprise an inorganic material as described below (e.g., ceramic or metal).

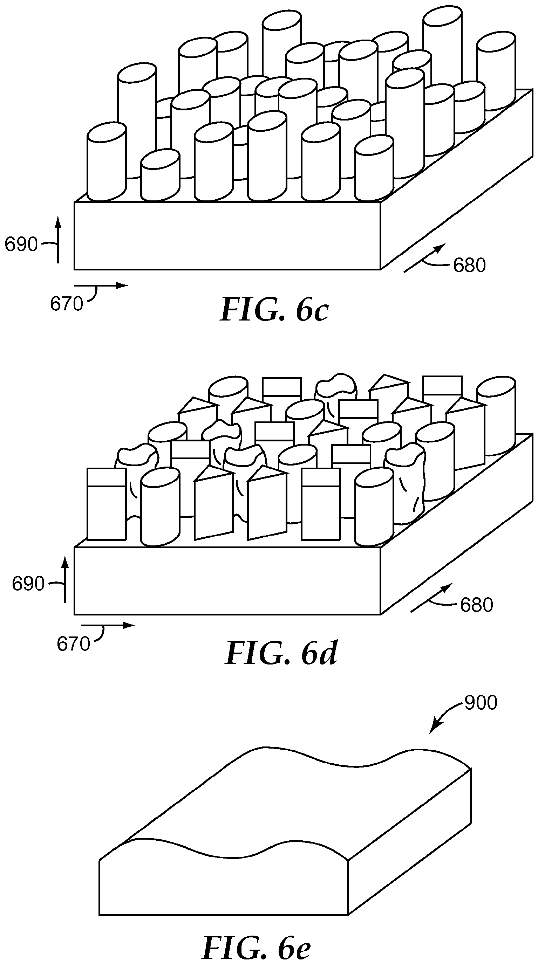

Referring again to Figure 1, engineered surface 110 of the medical article 100 comprises a substrate. The substrate may planar, substantially planar, or included varying topography (e.g., undulations) as depicted in Figure 6e. The substrate may be made from any number of suitable materials typically used to form medical articles. The substrate can be formed from a metal, alloy, polymer, biologic scaffolding, or a combination comprising at least one of the foregoing. The thickness of the substrate can vary depending on the use of the medical article. For example, in some embodiments, the substrate may be made from the same materials as microstructures 102, including those described above. In such embodiments, the substrate may be a material that is a majority silicone polymer by weight. In one exemplary embodiment, the substrate may be made of PDMS. In other exemplary embodiments, the substrate may be made of other commonly used substrates. Specifically, glass, ceramic, metal or polymeric substrates may be appropriate, as well as other suitable alternatives and combinations thereof such as ceramic coated polymers, ceramic coated metals, polymer coated metals, metal coated polymers and the like.

Biocompatible metals for use as the substrate include stainless steel alloys such as type 316 L, chromium-cobalt-molybdenum alloys titanium alloys such as TiAl₄V, zirconium alloys, shape memory nickel-titanium alloys, super elastic nickel-titanium alloys, and combinations thereof.

The polymers used to form the substrate can be biodegradable, non-biodegradable, or combinations thereof. In addition, fiber- and/or particle-reinforced polymers can also be used. Non-limiting examples of suitable non-biodegradable polymers include polyisobutylene copolymers and styrene-isobutylene-styrene block copolymers, such as styrene-isobutylene-styrene tert-block copolymers (SIBS); polyvinylpyrrolidone including cross-linked polyvinylpyrrolidone; polyvinyl alcohols; copolymers of vinyl monomers such as EVA; polyvinyl ethers; polyvinyl aromatics; polyethylene oxides; polyesters such as polyethylene terephthalate; polyamides; polyacrylamides; polyethers such as polyether sulfone; polyolefins such as polypropylene, polyethylene, highly crosslinked polyethylene, and high or ultra high molecular weight polyethylene; polyurethanes; polycarbonates; silicones; and siloxane polymers. Certain suitable natural based polymers may or may not be biodegradable includeoptionally modified polysaccharides and proteins including, but not limited to, cellulosic polymers and cellulose esters such as cellulose acetate; and combinations comprising at least one of the foregoing polymers. Combinations may include miscible and immiscible blends as well as laminates.

Non-limiting examples of suitable biodegradable, bioabsorbable, bioerodable, or bioadhesive polymers include polycarboxylic acid; polyanhydrides such as maleic anhydride polymers; polyorthoesters; poly-amino acids; polyethylene oxide; polyphosphazenes; polylactic acid, polyglycolic acid, and copolymers and mixtures thereof such as poly(L-lactic acid) (PLLA), poly(D,L,-lactide), poly(lactic acid-co-glycolic acid), and 50/50 weight ratio (D,L-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-blutylacrylate; polyhydroxybutyrate valerate and mixtures thereof; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid, cellulose, and hydroxypropylmethyl cellulose; gelatin; starches; dextrans; and alginates and derivatives thereof, proteins and polypeptides; and mixtures and copolymers of any of the foregoing. The biodegradable polymer can also be a surface erodable polymer such as polyhydroxybutyrate and its copolymers, polycaprolactone, polyanhydrides (both crystalline and amorphous), and maleic anhydride.

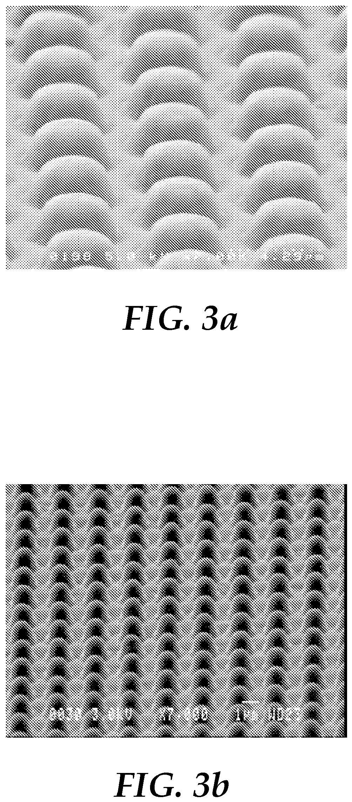

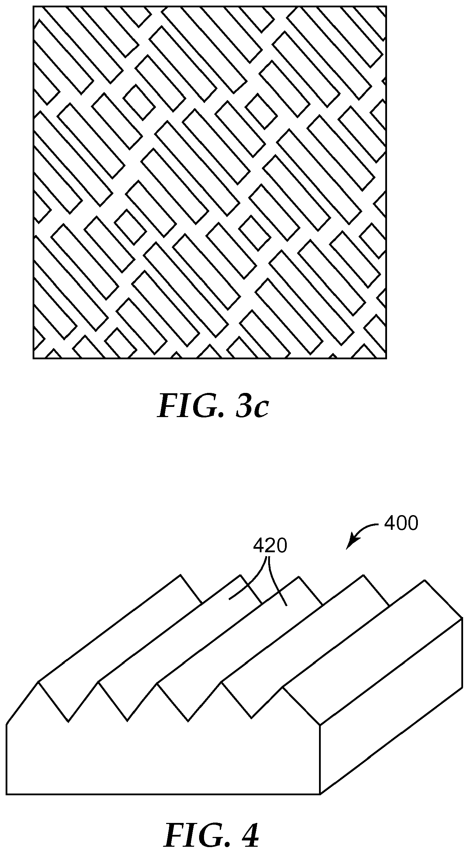

Although Figure 1 illustrates dome-shaped engineered structures 120, a number of different shapes are contemplated. For example, as illustrated in Fig. 3a-d, engineered structures may be dome-shaped as illustrated in SEM image 3a, post-shaped, as shown on SEM image in Fig. 3b, a pattern that reportedly mimics shark skin, as shown in Fig. 3c, or prism-shaped, as shown in Fig. 3d. A wide variety of additional shapes are also contemplated.

Further examples of suitable engineered structure shapes can include, but are not limited to, a variety of polyhedral shapes, parallelepipeds, prismatoids, prismoids, etc., and combinations thereof. For example, a structure can be polyhedral, conical, frusto-conical, pyramidal, frusto-pyramidal, spherical, partially spherical, hemispherical, ellipsoidal, dome-shaped, cylindrical, and combinations thereof.

Additional suitable shapes include irregular geometries that can be described by non-Euclidean mathematics. Non-Euclidean mathematics is generally used to describe those structures whose mass is directly proportional to a characteristic dimension of the spaced feature raised to a fractional power (e.g., fractional powers such as 1.34, 2.75, 3.53, or the like). Examples of geometries that can be described by non-Euclidean mathematics include fractals and other irregularly shaped microstructures.

Generally, engineered structures according to the present invention comprise a base 112 adjacent the engineered surface 110 and a top surface 108 separated from base 112 by a height 114. It should be appreciated that the terms "height" , "base" and "top" are for used illustrative purposes only, and do not necessarily define the relationship between the surface and the structure. For example, the "height" of a microstructure projected into a surface can be considered the same as the depth of recess created, and the "top surface" the bottom of said recess.

The base 112 of each engineered structure 120 may comprise a variety of cross-sectional shapes including, but not limited to, parallelograms, parallelograms with rounded corners, rectangles, squares, circles, half-circles, ellipses, half-ellipses, triangles, trapezoids, stars, other polygons (e.g., hexagons), etc., and combinations thereof.

Regardless of cross-sectional shape, each engineered structure comprises a smallest cross-sectional dimension at the base 112. In certain implementation, the smallest cross-sectional dimension of the base can be no greater than 20 microns, in some embodiments no greater than 10 microns, and in some embodiments no greater than 5 microns. The smallest cross-sectional dimension may be at least 0.1 microns, in some embodiments at least 0.5 microns, and in some embodiments at least 1 micron. In other implementations, the smallest cross-sectional dimension may be no greater than 50 microns, in some implementations no greater than 25 microns, and in some implementations no greater than 15 microns.

In certain implementations, no cross-sectional dimension at the base 112 exceeds 5 microns. Engineered structures having cross-sectional dimensions no greater than 5 microns arranged according to the present disclosure are believed to substantially interfere with the settlement and adhesion of target bacteria most responsible for HAIs or other bio fouling problems such as increased drag, reduced heat transfer, filtration fouling, etc.

Generally, each engineered structure or the plurality of engineered structures has a height 114 that is at least 0.5 microns, however shorter microstructures are contemplated (e.g., 200 nm). In some embodiments, each microstructure of the plurality of microstructures has a height of at least 1 micron, in other embodiments at least 1.5 microns, in other embodiments at least 2 microns, in other embodiments at least 3 microns and in other embodiments at least 5 microns.

In certain embodiments, the engineered structure height is no greater than 100 microns, in some embodiments no greater than 50 microns, in some embodiments no greater than 30 microns, in some embodiments no greater than 20 microns, and in certain preferred embodiments no greater than 10 microns.

Whether protruding from or projecting into the engineered surface, each engineered structure of the plurality of engineered structures includes a particular aspect ratio. For engineered structures comprising regular (e.g., Euclidean) and irregular (e.g., Non-Euclidean) cross-sectional shapes substantially throughout the height of the microstructure, the aspect ratio is defined herein as the ratio of the height to the smallest cross-sectional dimension (e.g., width, length, diameter) at the base. For irregularly shaped bases (bases which are not parallelograms or circles) the smallest cross-sectional dimension will be understood to be the diameter of a circle of equivalent area. Regardless of structure geometry, each engineered structure includes an aspect ratio of at least 0.1, in some embodiments at least 0.5, and in some embodiments at least 1. The aspect ratio of each engineered structure may be no greater than 15, and in some embodiments no greater than 10.

In certain preferred embodiments, the plurality of engineered structures comprises an array of posts as depicted in Figure 3b. The base 112 of the post may comprise a variety of cross-sectional shapes including, but not limited to, parallelograms, parallelograms with rounded corners, rectangles, squares, circles, half-circles, ellipses, half-ellipses, triangles, trapezoids, stars, other polygons (e.g., pentagons, hexagons, octagons), etc., and combinations thereof.

In certain embodiments, the cross-sectional area of the post does not substantially change in a vertical direction as the top surface 108 is approached. For example, if the base comprises a circular cross-section, the diameter will not substantially change between the top surface and the base. In other embodiments, the cross-sectional area may increase or decrease in a vertical direction relative to the engineered surface.

Typically, the top surface of the post is substantially planar and is substantially parallel to the engineered surface. It is further contemplated, however, that the top surface include at least a portion that is concave or convex, such that the post appears rounded or dimpled on the top surface. Though not depicted, the top surface of the post can include additional features having one of the myriad shapes discussed above.

Where the engineered structures 420 are prism-shaped as in Figure 3c, the pyramids may have a peak angle (or angle between the two facets of a prism) of 90 degrees. As the prism-shaped structures 420 are depicted are isosceles triangles, the angle of intersection of the two facets with the plane of the film will then be an angle of 45 degrees. In other embodiments, the peak angle may be greater or less than 90 degrees. For example, the peak angle may be between 90 degrees and 100 degrees, or between 80 degrees and 90 degrees.

Referring again to Figure 1, the nanofeatures 140 are formed into or onto the engineered structure 120 and preferably cover a great deal of the surface of the structure. It should be appreciated that nanofeatures 140 are not drawn to scale in relation to engineered structures 120. The nanofeatures 140 may generally have an average width of between about 5nm to about 250nm, in certain preferred implementations between 25 nm and 150nm. The nanofeatures 140 generally have an average height of between about 5 nm and about 1000nm, potentially between about 20 nm and about 500 nm, in some embodiments between 30 nm and 100 nm, and in other embodiments between 30 nm and 75 nm. As such, nanofeatures 140 can include a high aspect ratio in a number of implementations. Each nanofeature includes a particular aspect ratio. For nanofeatures comprising regular (e.g., Euclidean) and irregular (e.g., Non-Euclidean) cross-sectional shapes substantially throughout the height of the microstructure, the aspect ratio is defined herein as the ratio of the height to the smallest cross-sectional dimension (e.g., width, length, diameter) at the base. For irregularly shaped bases (bases which are not parallelograms or circles) the smallest cross-sectional dimension will be understood to be the diameter of a circle of equivalent area. In some embodiments, the nanofeatures exhibit an average aspect ratio of at least about 1 to 1, or at least about 2 to 1, or at least about 3 to 1, or at least about 4 to 1, or at least about 5 to 1, or at least about 6 to 1. In other embodiments, the average aspect ratio may be on the order of 1 to 2.

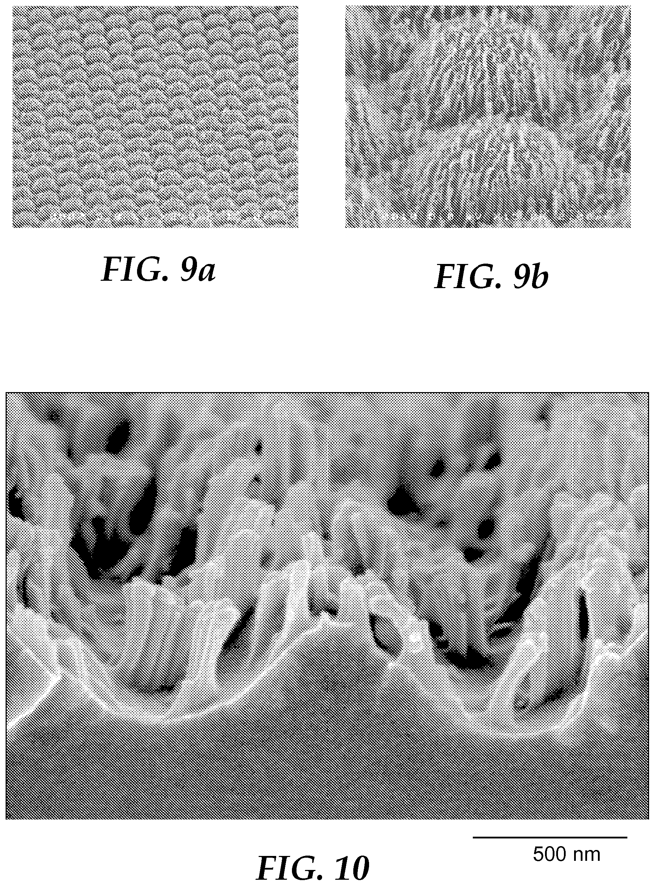

In certain implementations, individual nanofeatures may converge during formation to form unitary structures composed of several nanofeatures. (See e.g, Figure 10). Certain characteristics of individual nanofeatures may or may not be distinguishable in such structures. These unitary structures are still appropriately considered nanofeatures, however, and the aspect ratios of such structures will be governed by the preceding paragraph. For example, the clustered nanofeatures may have a width of about 200 nm and a height of about 300 nm.

In implementations featuring recessed engineered structures, the directed nanofeatures can be projected further into the surface. In other embodiments, the nanofeatures protrude from the surfaces of the recess. One exemplary method of making nanofeatures projecting into a surface is to provide a mask with holes to expose the substrate in spots. This then can be exposed to reactive ion etching to produce a nanofeature projecting into the substrate.

In certain preferred implementations, the nanofeatures are not deliberately patterned on the surface of the microstructures. Randomly distributed nanofeatures have been found to enhance the anti-adhesive properties of engineered microstructures and can be easier to create.

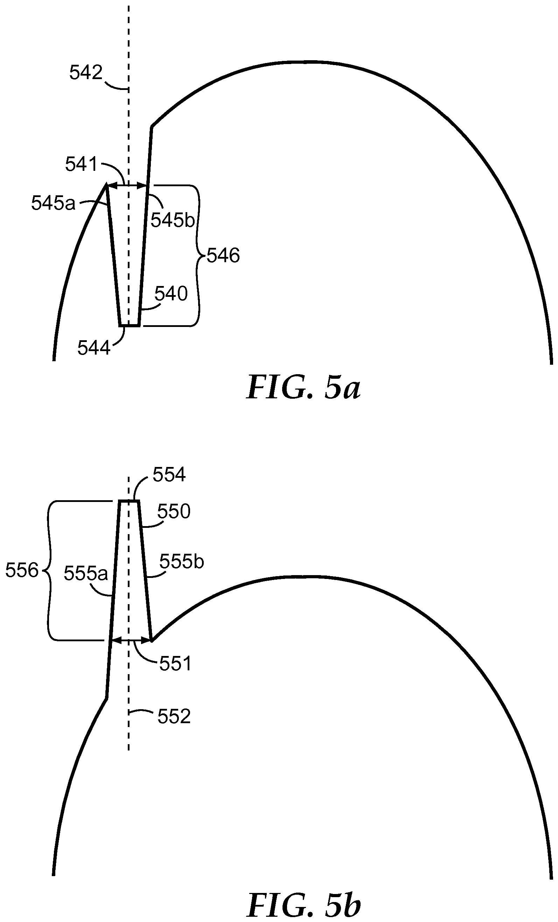

Nanofeatures may be created as nanocavities and nanoposts, as depicted in Figs. 5a-b. Referring to Fig. 5a, nanocavity 540 has a width 541 that may be understood as the maximum width of the nanocavity 540 in a plane perpendicular to the cavity axis 542 that is furthest from the base of the cavity 544 and still intersects all side walls 545a and 545b of the cavity. The length 546 of the nanocavity is the distance to this same point along the cavity axis from the base of the cavity 544. Dividing the length 546 by the opening width 541 provides the length-to-opening ratio of a given nanocavity 540. By adding all of the length-to-opening ratios and dividing by the number of nanocavities of the engineered surface, the average length-to-opening ratio may be determined. In some embodiments of according to the present disclosure, the average length-to-opening ratio may be at least 1 to 1, at least 3 to 1, or at least 5 to 1, or 10 to 1 or 15 to 1, or even at least 20 to 1.

Similarly and as shown in Fig. 5b, nanopost 550 has a width 551 at its base that may be understood as the maximum width of the nanopost 550 in a plane perpendicular to the structure axis 552 that is furthest from the peak 554 of the structure and still intersects all side walls 555a and 555b of the nanopost. The length 556 of the nanopost is the distance to this plane along the structure axis from the peak of the structure 554. Dividing the length 556 by base width 551 provides the length-to-base ratio of a given nanopost 550. By adding all of the length-to-base ratios and dividing by the number of nanoposts protruding from the surface, the average length-to-base ratio may be determined. In some embodiments according to the present description, the average length-to-base ratio may be at least 3 to 1, or at least 5 to 1, and in some the length-to-base ratio may be at least 10 to 1, or 15 to 1, or even at least 20 to 1.

The nanocavities and nanoposts may have different shapes, especially at the deepest portion of the cavity and top of the post. For example, in some aspects, the nanocavity and/or nanopost may be shaped in a near rectangular shape, or at least a trapezoid, such that the bottom/top of the nanocavity/nanopost is flat and does not terminate at a point. In other embodiments, the cavity or post may terminate at a single point, such that the cavity or post is a rectangle shape that is needle-like. Either shape of nanocavity or post may be characterized by their length-to-opening/length-to-base ratio to demonstrate the whisker-like structure thereof.

Although the nanocavities and nanoposts described thus far are generally illustrated as having smooth, or nearly smooth sidewalls, other constructions are also contemplated. The walls of the nanoposts or nanocavities also may have a certain level of roughness. The roughness may in fact be substantial enough that the side walls of the nanoposts or nanocavities appear to be wavy in shape.

As can be appreciated, the engineered structures described above may be considered a first-level feature. In certain embodiments, the first level feature includes at least one microscale dimension (i.e., the smallest dimension may be on the nano-scale, but, for example, width or height may be a least one micron). In other embodiments, the first level feature includes no dimension greater than 0.99 microns and no dimension less than 0.1 microns. In such embodiments, the engineered surfaces comprises a first level of nanofeatures having one set of dimension and a second level of nanofeatures having dimensions less than smallest dimension of the nanofeatures on the first level. For example, the first level can comprise dome-shaped features having a width of 600 nm and a height of 200 nm. The second level of this exemplary surface can comprise nanofeatures having a height of 130 nm to 330nm and a width of 40 nm to 70 nm.

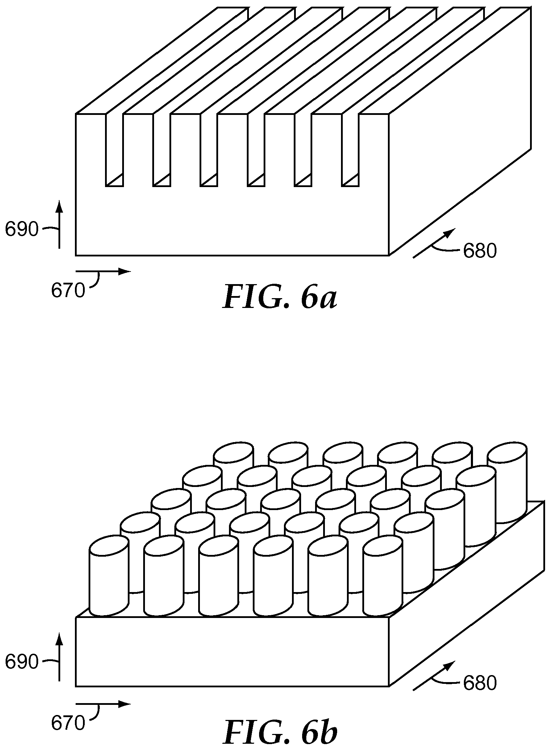

Turning to Figures 6a - 6d, the plurality of microstructures may be arranged on an engineered surface according to any number of patterns. The engineered surface may be planar (as depicted in Figures 6a-6d), substantially planar, or included varying topography (e.g., undulations) as depicted in Figure 6e. More generally, microstructures may be created that vary in one, two or three dimensions. Further, the engineered structures may be continuous or discontinuous. For example in Fig. 6a, the engineered structures may be continuous structures that identically run a length 680 of the film at the same height along the vertical direction 690 without any segmentation. However, across the width 670 of the portion of the engineered surface, or across a first dimension, the film is segmented into different discrete microstructures. In addition, as shown in Fig. 6b, the engineered structures may vary in two directions, such that the surface comprises discontinuous engineered structures. For example, the engineered structures may be segmented as in Fig. 6a along the width 670 (i.e., the x-axis) of a portion of the engineered surface, but also be segmented along the length 680 (i.e., y-axis) of that portion of the engineered surface. In such a case, discrete posts are located along both the x and y axes. Here, however, the structures are all the same height in the vertical direction (or third dimension) 690. Further, as shown in Fig. 6c, the structures may be segmented along both the width and length of the surface and may also vary in the height of the microstructures across the surface in the z-axis (or third dimension). Finally, the engineered surface may comprise a plurality of engineered structure shapes, including combinations of regular and irregular engineered structures in any of the patterns described above. In any of these scenarios the engineered structures may be directly adjacent to one another or may be spaced apart by some portion of the engineered surface that is substantially flat.

Orientation of the engineered structures can be important with regard to the intended use of the medical article that includes the engineered surface. Orientation, aspect ratio, and material compliance/deformability of a microstructure may be considered relative to the engineered surface, the neighboring microstructures, or both. Structures such as that shown in Fig. 6a have very different frictional properties in the two major directions (670 and 680). For example, it may be beneficial to have the lower frictional direction oriented along the length of a catheter (urinary or venous) or an endotracheal tube.

As illustrated by certain previous figures herein (e.g., Fig. 4), the engineered structures may be directly adjacent to one another, such that the base of an engineered structure is directly in contact with the base of an adjacent engineered structure. However, it should be understood that the engineered structures may be further spaced apart, such that the facets or perimeters of the engineered structures are not in contact and are spaced apart by a segment of engineered surface that may, for example, be flat. The configuration of engineered structures in any given region should be chosen, however, so that the average pitch (i.e., the centroid to centroid distance between adjacent engineered structures) is at least as large as the smallest dimension of the smallest engineered structure and no greater than 5 times the smallest dimension of the engineered structure. Surfaces having pitches outside this range may result in topographies that reduce bioadhesion in certain areas, but at least fail to impede or potentially promote bioadhesion in other areas.

In certain embodiments, the engineered structures are segmented in two dimensions, preferably the along the x-axis and the y-axis (i.e., the height of each microstructure along the z-axis is substantially the same). The average pitch may be the same in both dimensions. In other embodiments, the pitch along the x-axis is less than the pitch along the y-axis, and vice versa.

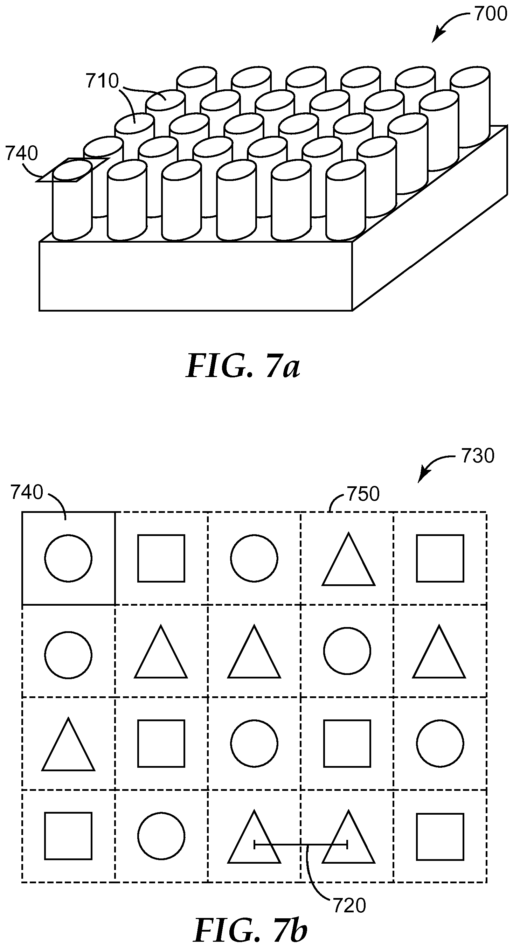

The arrangement of engineered structures on the engineered surface can comprise a particular density of engineered structures per square centimeter. In some embodiments, the engineered surface comprises at least 16 structures per square centimeter, in some embodiments, at least 64, and in yet other embodiments at least 400 structures per square centimeter. The engineered surface may comprise no greater than 4,000,000 engineered structures per square centimeter, in some embodiments no greater than 1,000,000, in some embodiments no greater than 160,000, in some embodiments no greater than 40,000, and in other embodiments no greater than 1000 structures/cm². Without wishing to be bound by theory, surfaces with engineered structure densities less than 16 and greater than 4,000,000 may not sufficiently disrupt biofilm formation as such surfaces may provide greater area for attachment and are believed to act essentially as a flat surface. As can be appreciated by reference to Figures 7a-f, the arrangement of engineered structures 710 on at least a portion of the engineered surface 700 comprises a plurality of unit cells740. Each unit cell 740 of the plurality of unit cells 730 exists in a single plane (i.e., two dimensions), is at least partially defined by a dimension at least approximating the pitch, and contains one microstructure. In certain embodiments, the unit cell is at least partially defined by a dimension equal to the pitch. As depicted in Fig. 7a, each unit cell 740 is entirely defined by the pitch between adjacent structures. Each unit cell includes a boundary 750 defining the perimeter of the unit cell. Each boundary 750 is directly adjacent the boundary of a neighboring unit cell, so that the plurality of unit cells resemble, e.g., a grid or tessellation.

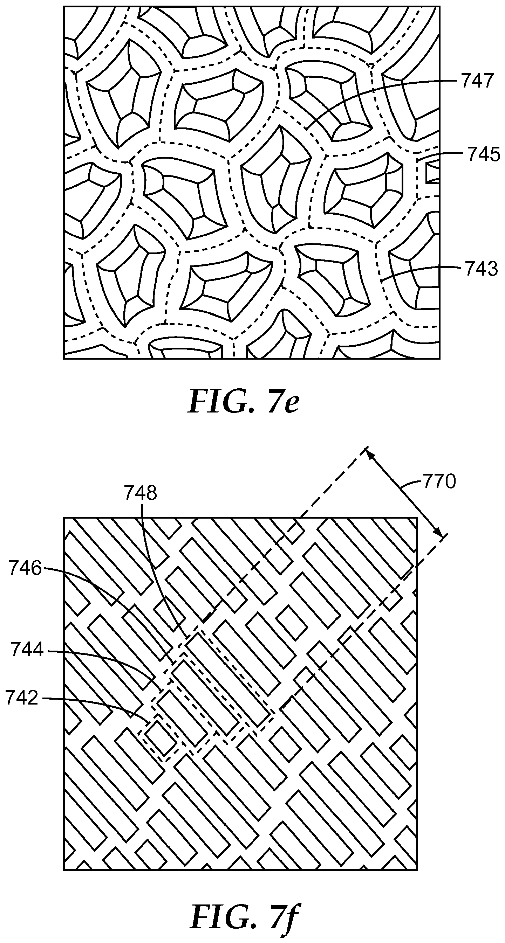

In other embodiments, as depicted in Fig. 7f, unit cells can include certain dimensions that are greater than the pitch. As can be appreciated, the length 770 of unit cell 748 is greater than the pitch 720.

In certain embodiments, a single unit cell geometry is repeated over at least a portion of the engineered surface 700. Preferably, each unit cell includes no more than one engineered structure and all structures of the plurality of engineered structures 740 share the substantially same or same geometry and/or orientation. In certain advantageous embodiments, such as the one depicted in Fig. 7a, at least a portion of the engineered surface comprises posts having the same geometry and orientation.

In another embodiment depicted in Fig. 7b, at least a portion of the engineered surface 700 comprises a plurality of structure geometries. For example, a unit cell including a pyramidal projections is directly adjacent a unit cell including a post. Each unit cell is still defined at least partially by a dimension at least approximating, preferably equal to, the pitch and includes a single microstructure.

A variety of shapes may be used to define the unit cell around a single engineered structure. Suitable unit cells may comprise rectangles, circles, half-circles, ellipses, half-ellipses, triangles, trapezoids, and other polygons (e.g., pentagons, hexagons, octagons), etc., and combinations thereof. Regardless of regular unit cell shape, the boundaries of any unit cell are directly adjacent the boundary of a neighboring unit cell and the unit cell is at least partially defined by a dimension equal to the pitch, such that the arrangement resembles a tessellation.

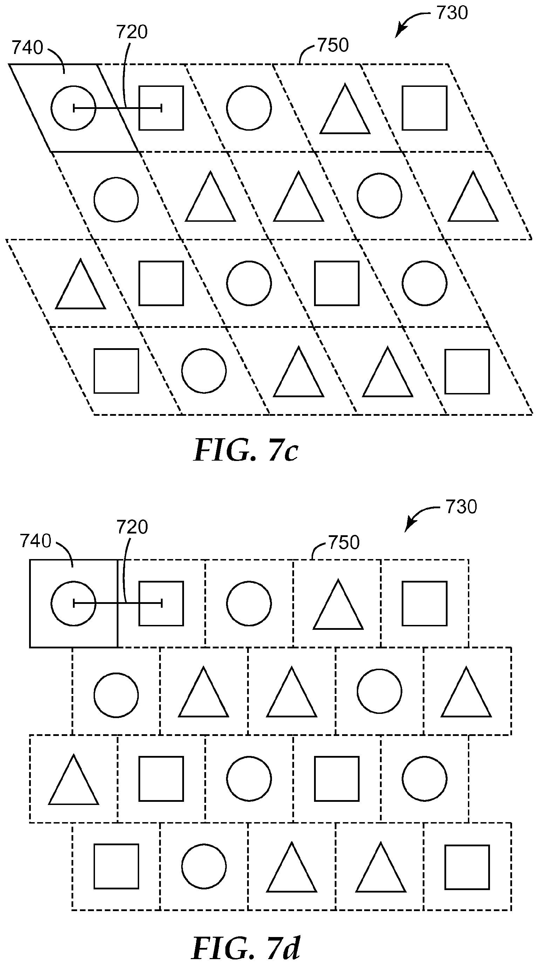

The arrangement of unit cells on any portion (or entire portion) of the engineered surface can resemble a structured or unstructured grid array. Exemplary structured arrays or tessellations include a Cartesian grid array as depicted in Figs. 7a-b and a regular grid array as depicted in Fig. 7c. In other embodiments, the arrangement of unit cells resembles a tessellation as depicted in Fig. 7d, with adjacent unit cells offset or alternating in one dimension. An exemplary unstructured array featuring three unique unit cell geometries (743, 745, and 747) is depicted in Figure 7e.

In certain embodiments, at least a portion of the engineered surface includes no more than three unique unit cell geometries. In other embodiments, at least a portion of the engineered surface includes no more than two unique unit cell geometries. In certain implementations, at least a portion of the engineered surface includes only one unit cell geometry repeated over said surface portion. As described above, the unit cell in such embodiments can contain engineered structures having substantially similar geometry or microstructures having a different geometry. Engineered surfaces having more than three distinct unit cell geometries, as shown in Figure 7f as unit cells 742, 744, 746, and 748, render replication of the microstructures substantially more difficult due to e.g., shrinkage of material and mold replication difficulties.

In yet other embodiments, at least portions of the engineered surface may be defined by irregular unit cells containing one or more structure geometries and/or orientations. The unit cell in such embodiments is not necessarily at least partially defined by the pitch (but can be in certain embodiments). For example, the irregular unit cell may include the smallest dimension that is shared by one or more adjacent structures or by two adjacent unit cells. As another example as depicted in Figure 7e, the irregular unit cells can be drawn around the base shape of the engineered structure and still resemble a tessellation. The structures of such irregular unit cells can have variety of geometries and can exist in one, two or three dimensions or any dimensions therebetween. The engineered structures in such unit cells can have similar geometries with different dimensions, similar geometries with similar dimensions, or can have different geometries with different dimensions.

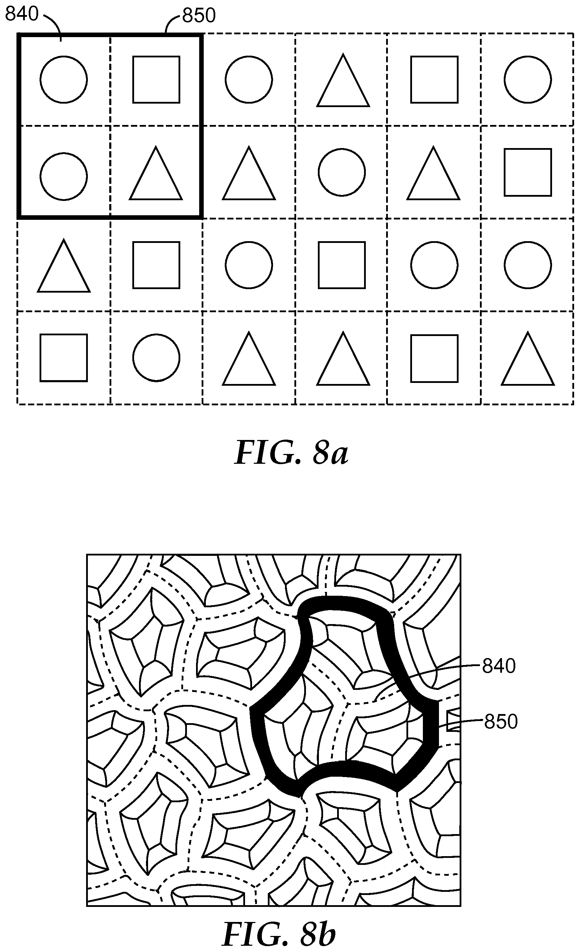

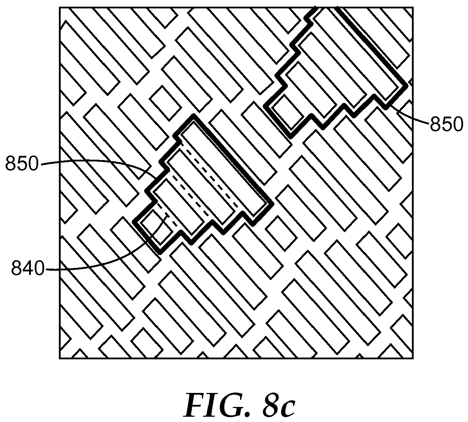

As depicted in Figures 8a and 8b, when a plurality of regular or irregular unit cells are arranged as a tessellation according to the present disclosure, multiple unit cells 840 can be grouped into larger, mother cells 850. The mother cells 850 are also arranged in a tessellation such that the boundaries of any mother cell 850 are directly adjacent the boundary of a neighboring mother cell. In contrast, and as depicted in 8c, certain arrangements of microstructures including four (4) or more distinct unit cell geometries include mother cells 850 that cannot be tessellated.

In a different aspect, the present disclosure relates to a method of producing anti-adhesion surfaces. In such embodiments, the engineered surface 110 can be formed by a variety of methods, including a variety of microreplication methods, including, but not limited to, casting, coating, and/or compressing techniques. For example, first level structuring of the engineered surface 110 can be achieved by at least one of (1) casting a molten thermoplastic using a tool having a structured pattern, (2) coating of a fluid onto a tool having a structured pattern, solidifying the fluid, and removing the resulting film, (3) passing a thermoplastic film through a nip roll to compress against a tool having a structured pattern (i.e., embossing), and/or (4) contacting a solution or dispersion of a polymer in a volatile solvent to a tool having a structured pattern and removing the solvent, e.g. by evaporation. The tool can be formed using any of a number of techniques known to those skilled in the art, selected depending in part upon the tool material and features of the desired topography. Illustrative techniques include etching (e.g., chemical etching, mechanical etching, or other ablative means such as laser ablation or reactive ion etching, etc., and combinations thereof), photolithography, stereolithography, micromachining, knurling (e.g., cutting knurling or acid enhanced knurling), scoring, cutting, etc., or combinations thereof.

Alternative methods of forming the engineered surface 110 include thermoplastic extrusion, curable fluid coating methods, and embossing thermoplastic layers, which can also be cured. Additional information regarding the substrate material and various processes for forming the engineered surface 110 can be found, for example, in Halverson et al., PCT Publication No. WO 2007/070310 and US Publication No. US 2007/0134784; Hanschen et al., US Publication No. US 2003/0235677; Graham et al., PCT Publication No. WO2004/000569; Ylitalo et al., US Patent No. 6,386,699; Johnston et al., US Publication No. US 2002/0128578 and US Patent Nos. US 6,420,622, US 6,867,342, US 7,223,364 and Scholz et al., US Patent No. 7,309,519 .

With microreplication, the engineered surface 110 can be mass produced without substantial variation from product-to-product and without using relatively complicated processing techniques. In some embodiments, microreplication can produce an engineered surface that retains an individual feature fidelity during and after manufacture, from product-to-product, that varies by no more than about 50 microns. In some embodiments, the engineered surface 110 retains an individual feature fidelity during and after manufacture, from product-to-product, which varies by no more than 5 microns, in some embodiments no more than 0.5 microns, and in preferred embodiments no more than 0.2 microns. In some embodiments, the engineered surface 110 comprises a topography that has an individual feature fidelity that is maintained with a resolution of between about 5 microns and 0.05 microns, and in some embodiments, between about 2.5 microns and 1 micron.

Next, the directed nanofeatures can be created on the engineered surface according to a variety of techniques. In one embodiment a method of creating nanofeatures includes a layer of nanoparticles applied to at least a portion of the engineered surface 110. Generally, the nanoparticles may be made up of any material that is conducive to serving as an etch mask for the film. For example, the nanoparticles may be a slow or substantially non-etching metal such as gold, or certain metal oxides, e.g., indium tin oxide, ZrO₂, CeO₂, AbO₃, SiO₂, or TiO₂, just to name a few. The particles may be applied as part of a binder or coating suspension as desired to best disperse the particles on the surface 110. The nanoparticles may be applied to the engineered surface by any appropriate coating method, such as dip coating, roll coating, die coating, spray coating, spin coating, and the like. Sputtering techniques may also be used. Coating method, equipment, process conditions and compositions may be selected to achieve a substantially uniform coating over the engineered surface.

In the subsequent step, the engineered surface 110 is etched using the nanoparticles as an etch mask. One particularly useful etching method for the etching step is reactive ion etching. Dry etching techniques such as laser ablation or ion beam milling may also be used. The result of the etching step is a plurality of nanostructures that are located on the portions of the microstructures and any portions of surface in between them. The nanostructures may be broadly understood in the current description as either nanofeatures that protrude from the surface of microstructures or valleys that are etched into the surface of microstructures 120. Where the nanoparticles used are slow-etching they may create either nanostructure valleys or protrusions that have high aspect ratios, such as 2 to 1, 3 to 1, 4 to 1, 5 to 1, 6 to 1 or even greater.

The present disclosure contemplates additional methods for creating nanofeatures on the microstructures and engineered surface. In one implementation, a layer of nanoposts is specifically applied on top of the microstructures, such that the nanoposts protrude out from the microstructures. The nanoposts may be applied by any suitable technique that does not interfere with the orientation or structural integrity of the structures. One appropriate method of nanopost deposition is to apply a film that is pre-structured with nanoposts. Otherwise, the layer of nanoposts may also first be applied as a non-structured deposited coating. In this manner of application, an organic pigment coating is vacuum deposited on to the engineered structures. Next, the organic pigment coating is annealed at an elevated temperature, preferably in vacuum. A more thorough description of this particular method may be found in U.S. Patent No. 5,039,561. Other means for generating the nanoposts include use of physical vapor transport growth instead of vacuum vapor deposition, vacuum deposition of inorganic materials at high incidence angle, ion or rf sputter etching of polymers, semiconductors or alloys having differential sputtering rates, sputter etching of polymers using microisland masking, photolithography (UV and X-ray), and electron beam lithography, electrochemical etching of metals and electroplating of roughened metals, photofabrication on photopolymer surfaces, directional solidification and etching of eutectics, and vapor liquid solid (VLS) crystal growth.

The nanoposts may generally be formed in fairly close proximity to one another, such that the nanoposts have an average spacing of less than about 100nm from one another. More specifically, the nanoposts may have an average spacing of between about 20nm and about 100nm from the closest adjacent nanopost. The nanoposts may further have an average spacing of between about 40nm and about 60nm. In accordance with this, the nanoposts can be very densely applied, such that the nanoposts have a density on the surface of the microstructures of between about 3 billion/cm² to about 5 billion/cm².

In a subsequent step, the first substrate is coated in a curable composition and cured to create a negative of the first substrate. The negative of the first substrate is then separated from the first substrate, and the nanoposts remain embedded in the negative after separation.

Finally, after separating the negative from first substrate, the negative is etched. The material of the negative may be etched away at a rapid rate by an appropriate etching method, such as reactive-ion etching. However, the nanopost can be coated in a slow-etching metal resulting in a slower etch of the nanoposts initially embedded in negative. The etch therefore may result in an engineered substrate that has a plurality of microstructures and nanoposts, where the nanoposts are transformed from embedded to protruding by the etch process.

Another method of creating nanofeatures includes providing a first substrate having a first surface. On the first surface are a number of engineered structures created according to methods described above. In the next step, a layer of easily etched nanoposts are applied to the engineered structures. In many embodiments the surfaces between engineered structures also is etched resulting in nanofeatures in the regions between microstructures. Next, the first substrate is coated in a curable composition and the curable composition is cured to create a negative of the first substrate. The negative is next separated from the first film, with the nanoposts remaining embedded in the negative after separation.

As opposed to the process previously described, no metal coating over the nanoposts is contemplated. In the next step, the top surface of the negative is etched away by an appropriate etch method, such as reactive ion etching or wet etching. However, due to the material of nanoposts, the nanoposts etch away at an even faster rate. The result of this step is a negative that has a plurality of nanocavities formed into the surface of negative. The negative may then serve as the engineered surface and may be secured to a medical article.

Alternatively, the engineered surface including a plurality of engineered structures and directed nanofeatures may be provided as a film and affixed to the substrate. In such embodiments, the engineered structures and/or nanofeatures may be made of the same or different material as the substrate. Fixation may be provided using mechanical coupling, an adhesive, a thermal process such as heat welding, ultrasonic welding, RF welding and the like, or a combination thereof.

It may be desirable to adequate nanofeature distribution throughout the engineered surface. Without wishing to be bound by theory, certain nanofeatures creation processes can result in nanofeatures located substantially in the areas between engineered structures, with little coverage on the surface of the engineered structures. Depending upon e.g., the height of the nanofeatures and the pitch between adjacent engineered structures, such an engineered surface may essentially act a flat surface and do little to resist adhesion. Accordingly in certain preferred implementations, the directed nanofeatures are disposed both between and on the engineered structures.

As a final optional step, surface energy modifying coating may be applied to the engineered surface. For example, a low surface energy coating may be desired. A low surface energy coating may generally be understood as a coating that, on a flat surface, has a water contact angle of greater than 110 degrees. Such a coating may not be necessary to achieve highly hydrophobic performance. Exemplary low surface energy coating materials that may be used may include materials such as hexafluoropropylene oxide (HFPO), or organosilanes such as, alkylsilane, alkoxysilane, acrylsilanes, polyhedral oligomeric silsequioxane (POSS) and fluorine-containing organosilanes, just to name a few. A number of other suitable low surface energy coatings may also be used to further enhance the hydrophobicity of the film. Examples of particular coatings known in the art may be found, e.g. in US Publication No. 2008/0090010, and commonly owned publication, US Publication No. 2007/0298216. Where a coating is applied to the microstructures, it may be applied by any appropriate coating method, such as sputtering, vapor deposition, spin coating, dip coating, roll-to-roll coating, or any other number of suitable methods.

It also is possible and often preferable in order to maintain the fidelity of the engineered structures to put a surface energy modifying compound in the composition used to form the engineered structures. In this manner, the surface energy modifying compound is at least partially deposited on the surface of the engineered structure thereby modifying the surface energy. In certain instances it may be necessary to add one or more bloom additive compounds to enhance the mobility of the surface energy modifying compound in order to get it to the surface in sufficient amount. For example, the surface energy modifying compound may have greater solubility in the bloom additive than in the base structure composition. In other cases, the bloom additive may retard or prevent crystallization of the base composition. Suitable bloom additives may be found, for example, in International Publication No. WO2009/152345 to Scholz et al. and US Patent No. 7,879,746 to Klun et al.

Advantages of this invention are further illustrated by the following examples, but the particular materials and amounts thereof recited in these examples, as well as other conditions and details, should not be construed to unduly limit this invention. Unless otherwise indicated, all parts and percentages are by weight.

### EXAMPLES

### PREPARATIVE EXAMPLES A

### CREATING A FIRST LEVEL ENGINEERED SURFACE (MICROSTRUCTURES)

To fabricate the master for a microstructure, a photoresist (PR) pattern was fabricated on a silicon wafer by optical lithography. A 1500 nm thick layer of silicon dioxide was coated onto the PR microstructures by plasma-enhanced chemical vapor deposition (PECVD), using a Model PlasmaLab System 100, available from Oxford Instruments, Yatton, UK, using the parameters listed in TABLE 1. A fast deposition rate was used in order to create surface roughness.

**TABLE 1. Conditions used for depositing SiO₂ layer**

| Reactant/Condition | Value |
|---|---|
| SiH₄ | 300 sccm* |
| N₂O | 1600 sccm |
| N₂ | 600 sccm |
| Pressure | 1600 mTorr (=213 Pa) |
| Temperature | 60°C |
| High frequency (HF) power | 110W |

| | |
|---|---|
| *standard cubic centimeter | |

The master, as prepared above, was next adhered to a stainless steel disk using double-stick tape. It was then made conductive by plating a thin layer of silver. This was followed by electroforming a nickel layer from a sulfamate nickel bath at a temperature of 54.4°C (130° F) and using a current density of 18 ampere per square foot (ASF), which equals 197 ampere per square meter. The thickness of the resulting nickel deposit was about 51µm (0.02 inch). The fidelity of the microstructure pattern was maintained through these metal plating steps. After electroforming was completed, the nickel deposit was separated from the original silicon wafer master, and used as a mold to prepare polydimethyl siloxane (PDMS) replicates.

The Ni mold was then treated with a release agent consisting of 0.1 wt % HFPO (hexafluoropropyleneoxide) phosphonic acid delivered from a 49:1 HFE7100:IPA solution. The HFE7100 was supplied by 3M Company of St. Paul, MN as 3M™ Novec™ 7100 Engineered Fluid, methoxy-nonafluorobutane (C4F9OCH3),. The procedure for treating the mold was as follows:
1) The mold was cleaned in a plasma cleaner for 5 minutes.
2) The mold was dip coated in the 0.1 wt % HFPO release agent solvent solution.
3) The coated mold was heated in an oven for 1 hour at 120°C
4) After cooling, the mold was rinsed for 1 minute in fresh 0.1 wt % HFPO release agent solvent solution.

After this treatment, the mold was ready for use in the replication process. To prepare a polydimethyl siloxane (PDMS) replica of the microstructure, Sylgard 184 PDMS (available as SYLGARD 184 Silicone Elastomer Kit, available from Dow Corning, Midland, MI) and its curing agent were first thoroughly mixed in a 10:1 weight ratio. Air bubbles trapped in the mixture were removed by degassing for 30 min at low vacuum. The degassed mixture was then poured onto the Ni microstructure (mold) master, further degassed for another 30 min, and then cured on a hot plate at 80°C for 1 hour. After curing, the PDMS replica was peeled off the Ni mold. High-quality microstucture replicas of the mold were produced. FIGS. 3a-3b show the high quality fidelity of these engineered microstructure surfaces. A flat "control" surface of the same material was also prepared as Comparative Example 1.

All of the first level engineered (microstructure) surfaces used in the examples consisted of SYLGARD 184 polydimethylsiloxane (PDMS) and were created according to the methods describe above unless otherwise noted. The one exception was the 600 x 600 nm "Sawtooth" Film with 190 nm Nanoparticle (NP) deposted nanofeatures , described below. A description of the prepared Examples is summarized in TABLE 5.

### PREPARATIVE EXAMPLES B

### CREATING THE SECOND LEVEL ENGINEERED FEATURES ON FIRST LEVEL STRUCTURES (NANOSTRUCTURES/NANOFEATURES ON MICROSTRUCTURES)

### Preparation of Nanofeatures on a Parallel Triangular Rails Array Surface

A secondary level topography (nanostructure or nanofeature) was created on a base (first level) microstructure, which was first created by the methods described in Preparative Examples A, above. The base PDMS structure was Comparative Example 3, a PDMS surface formed into an array of parallel triangular rails with an 11 µm pitch between the peaks of the rails. The PDMS replica was first treated with O₂ plasma "O₂ flow: 40sccm, RF power: 75W, P: 65mTorr, time: 15s). This step was followed by a dip into a suspension of indium-tin oxide (ITO) nanoparticles (ITONPs, available from Advanced Nano Products Co., Ltd, Chungcheonbuk-do, Korea) in isopropanol (IPA) in a 1:100 volume ratio, at a coating speed of 65 mm per minute.

The sample with the coating of ITONPs was then etched by reactive ion etching, (RIE), Model PlasmaLab™ System100 available from Oxford Instruments, Yatton, UK) using the parameters listed in TABLE 2, below.

**TABLE 2. Conditions used for Reactive Ion Etching**

| Reactant/Condition | Value |
|---|---|
| C4F8 | 10-50 sccm |
| 02 | 0.5-10 sccm |
| RF power | 50-100W |
| Inductive Coupling Plasma (ICP) power | 500-2000 W |
| Pressure | 3-50 mTorr (0.4-6.7 Pascal |

| | |
|---|---|
| * standard cubic centimeter | |

After fabricating the nanofeatures by RIE, the sample was treated with a fluorinated silane (HFPO, 3M Company developed, US patent US 7,678,426). The procedure for treating the sample with HFPO was as follows: (1) Dip coat in 0.1 wt% HFPO in HFE7100, (2) Heat the coated sample on a hot plate at 120°C for 30 min.

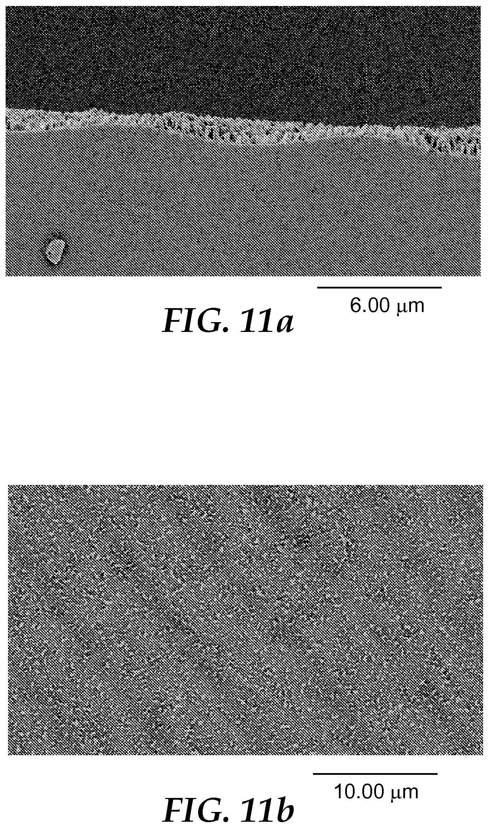

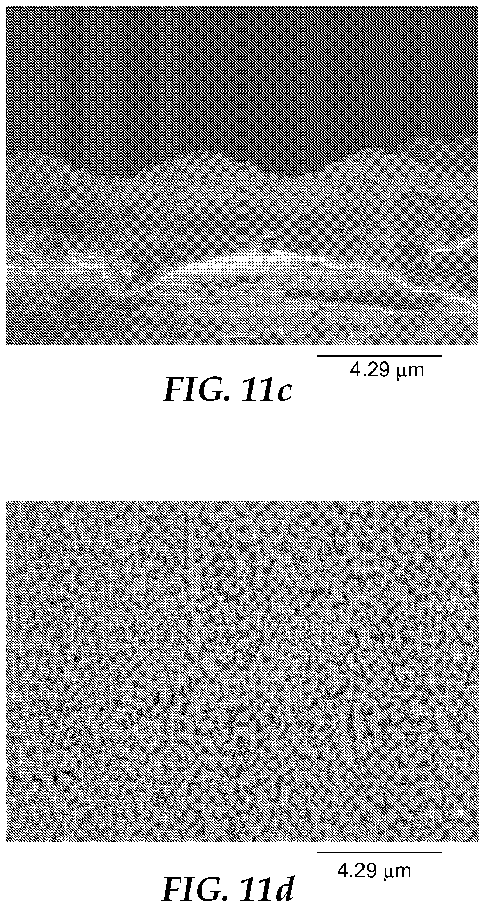

This was Example 4, which is shown in FIGS 11a-11b, where it can clearly be seen that uniform and dense nano-structures were formed on the array of parallel triangular rails with an 11 µm pitch.

Example 5 was prepared in the same with the same method as Example 4, above, with the exception that the base PDMS structure was an array of parallel triangular rails with a 6 µm pitch between the peaks of the rails. Example 5 is shown in FIGS. 1c-1d.

Comparative Example 2 was prepared by the same method as Example 4, above, with the exception that the base PDMS structure was a flat PDMS surface (Comparative Example 1), thus Comparative Example 2 was a flat surface with engineered nanofeatures.

### Preparation of Nanofeatures on Dome-Shaped Microstructured surfaces

A secondary level topography (nanostructure or nanofeature) was created on a base (first level) microstructure, which was first created by the methods described in Preparative Examples A, above. The PDMS base structure was Comparative Example 9, a PDMS dome-shaped pattern array with domes having (approximately) a 1.7 µm diameter, a 2 µm height with a pitch of 2.9 µm between domes. The nanofeatures were formed on the dome microstructures by following the same procedure described above for Example 4. The ITONPs were coated first, followed by Reactive Ion Etching (RIE), and finally treatment with HFPO. FIGS 9a-9b show SEM images of the resulting Example 10 with nanofeatures on the dome-shaped microstructure array. As can be seen in these micrographs, the nanofeatures have approximate dimensions of about 100-150 nm diameter with heights of approximately 500

### Preparation of Nanofeatures by RIE on a 600 x 600 nm Parallel Triangular Rails Array Surface

A secondary level topography (nanostructure or nanofeature) was created on a base (first level) structure, which was first created by the methods described in Preparative Examples A, above. The PDMS base structure was Comparative Example 6, an array of parallel triangular rails with rail heights of 600 nm and a pitch between the peaks of the rails of 600nm. The nanofeatures were formed on the array of 600 x 600 nm parallel triangular rails structures by following the same procedure described above for Example 4. The ITONPs were coated first, followed by Reactive Ion Etching (RIE), and finally treatment with HFPO.

### Preparation of 600 x 600 nm "Sawtooth" Film with 190 nm Nanoparticle (NP) deposted nanofeatures

**TABLE 3 Materials for Making 600 x 600 nm "Sawtooth" Film with 190 nm NP**

| Abbreviation/product name | Description | Available from |
|---|---|---|
| IRGACURE 184 | Photoinitiator | Ciba Specialty Chemicals, Tarrytown, NY |
| PHOTOMER 6210 | aliphatic urethane diacrylate | Cognis Corporation, Cincinnati, OH |
| SILQUEST A-174 | 3-methacryloxypropyltrimethoxysilane | Momentive Performance Materials, Inc., Friendly, WV |
| SR238 | 1,6 hexanediol diacrylate | Sartomer Company, Exton, PA |
| TX13112 | colloidal solution | Nalco Chemical Company, Naperville, IL |

A first level structure was fabricated to form a 600 nm pitch x 600 nm height "sawtooth" cross-section film by first making a multi-tipped diamond tool using focused ion beam (FIB) milling as described in U.S. Patent No. 7,140,812. The diamond tool was then used to make a micro-replication roll which was then used to make 600 nm 1D structures on a PET film in a continuous cast and cure process utilizing a polymerizable resin made by mixing 0.5% (2,4,6 trimethyl benzoyl) diphenyl phosphine oxide into a 75:25 blend of PHOTOMER 6210 and SR238. The resulting "sawtooth" first level engineered structure (repeating pattern) had a pitch of approximately 600 nm between the peaks of the parallel triangular rails and the structure and feature heights of approximately 600 nm.

### Preparation of A-174 modified 190 nm Silica

In a 500 mL flask, equipped with a condenser and a thermometer, 151.8 g of TX13112 colloidal solution (available from Nalco Chemical Company, Naperville IL), and 180 g of 1-methoxy-2-propanol were mixed together under rapid stirring. After that 1.48 g of Silquest A-174 was added. The mixture was heated to 80 °C, for 16 hours. After that 150 g of additional 1-methoxy-2-propanol was added. The resulting solution was allowed to cool down to room temperature. Most of water/1-methoxypropanol solvents were removed using a rotary evaporator under 60 °C water-bath, resulting in 59.73% by weight A-174 modified 190 nm silica dispersion in 1-methoxy-2-propanol.

**TABLE 4 Coating Formulation 1 (190nm NP)**

| Materials | % solids as supplied | Amount (grams) |
|---|---|---|
| A-174 modified 190 nm silica | 59.73% in PM | 15.9 |
| Ebecryl 8301 | 100 | 8.5 |
| HFPO-Urethane Acrylates | 30%wt in MEK | 7.2 |
| IPA (isopropanol) | 0 | 234 |
| 1-methoxy-propanol | 0 | 115 |
| Irgacure 184 | 100 | 0.35 |
| Total | | 380.7 |

The coating formulation 1, above, was prepared by mixing the entire gradient together under rapid stirring. The resulting solution was then applied on top of the 600 nm "sawtooth" film (600 nm pitch size) using a #10 wire-wound rod (obtained from RD Specialties, Webster, NY). The resulting films were then dried in air for 15 min, then cured using a Fusion UV-Systems Inc. Light-Hammer 6 UV (Gaithersburg, Maryland) processor equipped with an H-bulb, operating under nitrogen atmosphere at 75% lamp power at a line speed of 45 feet/min (2 passes).

**TABLE 5. Descriptions of Prepared Examples**

| Ex. | Example Surface Name | Type of Example: Comparative or Example | First Level (Microstructure) Description | Second Level (Nanofeature) | Related Figure |
|---|---|---|---|---|---|
| 1 | Flat PDMS | Comp. | PDMS flat | None | N/A |
| 2 | Flat PDMS w/ nano | Comp. | PDMS flat | YES - RIE created nanofeatures | N/A |
| 3 | 11µm Triangular Rails | Comp | PDMS surface of parallel triangular rails array with 11 µm pitch | None | FIG. 4 |
| 4 | 11 µm Triangular Rails w/nano | Example | Same as Ex. 3 | YES - RIE created nanofeatures | FIGS. 11a & 11b |
| 5 | 6 µm Triangular Rails w/nano | Example | PDMS surface of parallel triangular rails array with 6µm pitch | YES - RIE created nanofeatures | FIGS. 11c & 11d |
| 6 | 600 x 600 nm PDMS | Comparative | PDMS surface of parallel triangular | None | Similar in shape to |
| | "Sawtooth" | | rails array with 600 nm height and 600 nm pitch | | FIG. 4, but with smaller dimensions |
| 7 | 600 x 600 nm PDMS "Sawtooth" w/nano | Example | Same as Ex. 6 | YES - RIE created nanofeatures | FIG. 10 |
| 8 | 600 x 600 nm Rails Film with NP | Example | Acrylate Film surface with parallel triangular rails of 600 nm height and 600 nm pitch with "Sawtooth" like cross-section | YES - Nanoparticle (NP) deposited nanofeatures | FIG. 12 |
| 9 | Domes | Comparative | PDMS surface with array of dome shapes, 1.7µm diameter, 2 µm height and 2.9 µm pitch | None | FIG. 3a |
| 10 | Domes w/nano | Example | Same as Ex. 9 | YES - RIE created nanofeatures | FIGS. 9a & 9b |

### PREPARATIVE EXAMPLE C - BACTERIA SUSPENSIONS

### Preparation of S. aureus bacterial suspension

S. aureus bacteria were obtained from The American Type Culture Collection (Rockville, MD), under the trade designation ATCC 25923. The bacteria were grown overnight (17-22 hours at 37°C) in broth cultures prepared by inoculating 12 milliliters of prepared, sterile Tryptic Soy Broth (Hardy Diagnostics, Santa Maria, CA) with the bacteria.

### Preparation of P. aeruginosa, E. coli, and P. mirabillis bacterial suspensions

Additional bacterial suspensions were prepared in the same manner as the S. aureus suspension described above, for the following bacteria strains: P. aeruginosa bacteria (ATCC 15442), of P. aeruginosa ATCC (33494), E. coli bacteria (ATCC 700928) and P. mirabillis bacteria (ATCC 14153). Unless otherwise noted the ATCC 15442 strain of P. aeruginosa bacteria was used in the static biofilm assay evaluations, below.

### Preparation of Artificial Urine (AU)

The following solutions were prepared in separate bottles (all chemicals used were obtained from SIGMA-ALDRICH, St. Louis, MO): In preparing these solutions, water was first added to a bottle followed by addition of the solution salts and compounds under agitation. Salts and compounds were added slowly, in the order listed above for a given solution, until fully dissolved.
Solution #1: In 0.7L of double distilled water dissolve: 0.65g calcium chloride monohydrate, 0.65g magnesium chloride hexahydrate, 4.6g sodium chloride, 0.65 sodium citrate dihyrate, 1.6 potassium chloride, 1.0g ammonium chloride.
Solution #2: In 0.1L of double distilled water dissolve: 2.3g sodium sulfate, 0.02g sodium oxalate, 2.8g potassium phosphate monobasic.
Solution #3: In 0.1 L of double distilled water dissolve 1.1 g creatinine.
Solution #4: In 0.1L of double distilled water dissolve 25.0g urea.

The solutions were then autoclaved to ensure sterility. Under continuous agitation, first solution #2 was slowly added to solution #1, followed by slowly adding solution #3, and finally adding solution #4. Throughout this addition process the solution was visually monitored to ensure that all components remained fully dissolved. The final pH was adjusted to 5.4 by adding either hydrochloric acid to lower the pH or sodium hydroxide to raise the pH. To finish the preparation, the artificial urine solution was sterile filtered.

### STATIC BIOFILM ASSAY

### Process Steps for Static Biofilm Assay

1. To begin the assay (Day 0), the culture tube containing a select one of the bacterial suspensions as prepared above (S. aureus or P. aeruginosa, E. coli, P. mirabillis) was mixed well by vortexing for 30-60 seconds to mix and suspend the bacteria. An amount of 10 mL of the incubated bacterial suspension was then added to 90 mL of sterile Tryptic Soy Broth and vortexed. The bacterial concentration of this sample was approximately 1x10⁷ CFUs/mL.
2. To precisely quantify the bacterial concentration of the stock sample prepared in step 1. above, the sample was enumerated by performing serial ten-fold dilutions of the stock solution and plating on blood agar plates (Hardy Diagnostics, Santa Maria, CA) 100uL of the -4, -5, and -6 dilutions. The plates are incubated overnight at 37°C, and the number of colony formed units were counted the next day (Day 1) to determine the exact concentration in CFUs/mL of the stock sample prepared in step 1. The colonies are visible can be manually counted.
3. Using a 1.27cm (1/2-inch) diameter punch, sample coupons of the surfaces to be tested are punched out. The samples are rinsed in isopropyl alcohol and allow to dry in a biological hood.
4. The samples are then placed into a polycarbonate 24-well titer and identified by marking the lid of the titer plate. Each microstructure type was included in at least duplicates, or triplicates depending on the availability of the sample.
5. On "Day 0", 2mL of the bacterial suspension prepared in step 1 was added to each well of the titer plate. The plate was then incubated at 37°C overnight.
6. Every day, the titer plate(s) were removed from the incubator and placed on a rocker shaker for 1 minute. In a biological hood, the growth media was removed from each well.
7. For one set of samples (herein referred to as post-rinsed samples), 2mL of fresh TSB growth media were added to each well using a repeat pipette.
8. For a duplicate set of samples (herein referred to as "rinsed" samples), 2mL of sterile water were added to each well. A pipette was then used to rinse the samples by withdrawing and expelling the rinse water 5-6 times for each sample, and finally removing the rinse water. This wash procedure was repeated two more times for each sample, using fresh sterile water, resulting in a total of 3 washes per sample. Finally, 2 mL of fresh TSB growth media was added to each well using a repeat pipette.
9. The titer plate(s) where then placed on the rocker shaker for 1 minute, and returned to the incubator overnight.
10. On days 7 and 14, samples were removed for analysis. For all samples removed ("rinsed" and "post-rinsed"), a total of 3 washes using 2 mL of sterile water were performed according to the procedure described in step 8, above.
11. The removed and washed samples were placed into a new 24-well titer plate and allow to dry.
12. Once dry, the samples were stained according to the Gram-Staining Protocol, below.

### Gram-Staining Protocol

Samples from the Static Biofilm Assay described above were stained in preparation for analysis by microscopy using the following protocol:

### Fixing Samples:

1. The sample were completely air-dried prior to fixing.
2. A large beaker was filled with methanol (Alfa Aesar, Stock #19393) and using a pair of tweezers, each sample was submerged completely for 1 minute and 10 seconds.
3. The sample was removed from methanol and allowed to air dry on a clean surface.

### Gram-Staining S. aureus Samples

1. A 30 mL beaker was filled with approximately 8mL of Gram crystal violet stain (Becton Dickson Co.).
2. A second beaker was filled with approximately 8mL of stabilized Gram iodine (Becton Dickson Co. Franklin Lakes, NJ).
3. A third beaker was filled with approximately 8mL of Gram decolorizer (Becton Dickson Co.).
4. Using a pair of tweezers, the sample was first submerged completely into the Gram crystal violet stain for exactly 1 minute. After 1 minute, the sample was rinsed thoroughly under a slow and gentle running stream of deionized water.
5. The sample was then submerged completely in the stabilized Gram iodine for exactly 1 minute, followed by a rinse in deionized water as described above.
6. The sample was finally submerged completely in the Gram decolorizer for exactly 10 seconds, followed by a final rinse in deionized water.
7. Excess water was removed by blotting and the sample was allowed to air-dry on a clean surface.

### Gram-Staining P. aeruginosa Samples

1. A 30mL beaker was filled with approximately 8mL of Gram safrin stain (Becton Dickson Co.).
2. Using a pair of tweezers, the sample was first submerged completely into the Gram safrin stain for exactly 2 minutes. After 2 minutes, the sample was rinsed thoroughly under a slow and gentle running stream of deionized water.
3. Excess water was removed, and the sample was allowed to air-dry on a clean surface.

### Analysis of Sample Surfaces for Biofilm Formation

Determination of the surface area covered by a given organism for the samples resulting from the Static Biofilm Assay(above) was conducted by analyzing at least five micrographs for each sample. The micrographs were obtained using a Leica microscope (Model DM4000B, available from Leica Microsystems Inc., Bannockburn, IL) outfitted with a CCD camera. The micrographs used for analysis were taken at a magnification of 40x. At this magnification (40x), the field of view for the microscope setup was 345 µm x 290 µm. The fractional area of the surface covered by bacteria was determined using SigmaScan software (available from Systat Software Inc., San Jose, CA). This image analysis process involved taking a given micrograph, converting the image to a grayscale version, deriving an intensity histogram of the image, and setting an intensity threshold on the histogram to isolate the stained bacterial cells from the rest of the image. The software was then capable of automatically calculating the total pixel area of the image that comprises the stained cells. That area was then divided by the pixel size of the entire image to arrive at the fractional area covered by the bacteria. For each sample, a total of at least 5 fields (micrographs) were analyzed in this manner. The standard deviation (Std. Dev.) is calculated from the analysis of the 5 micrographs times the number of replicates. The data from each field was used to calculate an average value of the fractional area covered by the bacteria as well as the 1σ standard deviation (error).

**TABLE 6. % Surface Coverage by P. aeruginosa**

| Surface Name (replicate) | Example. or Compara -tive | First Level Structural Length Scale (µm) | 7 days % Surface Coverage by P. aer. | Std. Dev. | 14 days % Surface Coverage by P. aer. | Std. Dev. |
|---|---|---|---|---|---|---|
| Ex. 1 Flat (1) | Comp. | Infinite/0 | 39 | 10 | 80 | 15 |
| Ex. 1 Flat (2) | Comp. | Infinite/0 | 33 | 4 | 72 | 5 |
| Ex. 3 (1) | Comp. | 11 | - | - | 45 | 8 |
| Ex. 3 (2) | Comp. | 11 | - | - | 59 | 9 |
| Ex. 4 (1) w/nano | Ex. | 11 | - | - | 29 | 7 |
| Ex. 4 (2) w/nano | Ex. | 11 | - | - | 32 | 8 |
| Ex. 6 (600 nm Rails) | Comp. | 0.6 width | - | - | 83 | 16 |
| Ex. 7 (600 nm Rails w/nano) | Ex. | 0.6 width | - | - | 12 | 4 |
| Ex. 8 (600 nm Rails Film w/nano | Ex. | 0.6 width | - | - | 47 | 12 |

**TABLE 7. % Surface Coverage by S. aureus**

| Surface Name (replicate) | Example or Comparative | Structural Length Scale (µm) | 7 days % Surface Coverage by S.Aur | Std. Dev. | 14 days % Surface Coverag e by S.Aur. | Std. Dev. |
|---|---|---|---|---|---|---|
| Ex. 1 Flat (1) | Comp. | Infinite/0 | 35 | 6 | 67 | 6 |
| Ex. 1 Flat (2) | Comp. | Infinite/0 | 45 | 8 | 86 | 10 |
| Ex. 3 (1) | Comp. | 11 | - | - | 30 | 8 |
| Ex. 3 (2) | Comp. | 11 | - | - | 30 | 3 |
| Ex. 4 (1) w/nano | Example | 11 | - | - | 15 | 6 |
| Ex. 4 (2) w/nano | Example | 11 | - | - | 15 | 7 |

**TABLE 8. Rinsing Effect on % Surface Coverage by P. aeruginosa - 14 days**

| Surface Name (replicate) | Example or Comparative | Rinsed % Surface Coverage by P.aer. | Std. Dev. | Post- Rinsed % Surface Coverage by P.aer. | Std. Dev. | Rinsing Difference | Rinsing Difference (%) |
|---|---|---|---|---|---|---|---|
| Ex. 1 Flat (1) | Comp. | 72 | 5 | 84 | 6 | 12 | 117 |
| Ex.1 Flat (2)* | Comp. | 70 | 11 | 92 | 10 | 22 | 131 |
| Ex. 2 Flat w/nano | Comp | 78 | 8 | 77 | 5 | (1) | 101 |
| Ex. 3 Tri. Rails | Comp. | 45 | 8 | 59 | 9 | 14 | 131 |
| Ex. 4 w/nano | Example | 29 | 7 | 32 | 8 | 3 | 110 |
| Ex. 5 w/nano | Example | 14 | 3 | 21 | 7 | | |
| Ex. 9 Domes | Comp. | 18 | 5 | 24 | 6 | 6 | 133 |
| Ex. 10 Domes w/nano | Example | 10 | 4 | 13 | 4 | 3 | 130 |
| Ex. 7 (600 nm Rails w/nano)* | Example | 10 | 7 | 12 | 4 | 2 | 120 |

| | | | | | | | |
|---|---|---|---|---|---|---|---|
| * P. aeruginosa was ATCC 33494 | | | | | | | |

**TABLE 9. Rinsing Effect on % Surface Coverage by S. aureus - 14 days**

| Surface Name | Example or Comparative | Rinsed % Surface Coverage by S. aureus | Std. Dev. | Post-Rinsed % Surface Coverage by S. aureus | Std. Dev. | Rinsing Difference | Rinsing Difference (%) |
|---|---|---|---|---|---|---|---|
| Ex.1 Flat | Comp. | 67 | 7 | 77 | 6 | 10 | 113 |
| Ex. 2 Flat w/nano | Comp. | 74 | 7 | 83 | 6 | 9 | 112 |
| Ex. 3 Tri. Rails | Comp. | 30 | 3 | 33 | 9 | 3 | 109 |
| Ex. 4 w/nano | Example | 15 | 7 | 18 | 8 | 3 | 117 |
| Ex. 5 w/nano | Ex | 11 | 4 | 17 | 6 | | |
| Ex. 9 Domes | Comp. | 9 | 3 | 10 | 6 | 1 | 111 |
| Ex. 10 Domes w/nano | Example | 8 | 3 | 12 | 5 | 4 | 150 |
| Ex. 7 (600 nm Rails w/nano) | Example | 9 | 5 | 11 | 6 | 2 | 122 |

### Effect of Artificial Urine

The effect of artificial urine as a growth media in the formation of a biofilm on surfaces with engineered nanofeatures was evaluated. Artificial Urine, described above, was used as prepared in place of the fresh TSB in steps 7 and 8, for the Static Biofilm Assay. The results are reported in the TABLE 10, below.

**TABLE 10. % Surface Coverage after Artificial Urine 14 Days Post Rinsed**

| Surface Name | % Surface Coverage by E. coli ATCC 700928 | E. coli Std. Dev. | % Surface Coverage by P. aer ATCC 33494. | P. aer Std. Dev. | % Surface Coverage by P. mirabillis ATCC 14153. | P. mira. Std. Dev. |
|---|---|---|---|---|---|---|
| Ex.1 Flat (Comparative.) | 100* | 0* | 100* | 0* | 100* | 0* |
| Ex. 10 Domes w/nano (Example) | 14 | 7 | 16 | 5 | 28 | 10 |

| | | | | | | |
|---|---|---|---|---|---|---|
| *Total coverage | | | | | | |

### Effect of Bovine Serum Albumin added to Growth Media

The Static Biofilm Assay described above was followed with the exception that a 3% BSA in TSB was used instead of just TSB. The BSA in TSB solution was added to each well of the titer plate and at the appropriate steps in the assay procedure. The Bovine Serum Albumin (BSA) was obtained from from Sigma-Aldrich of Milwaukee WI. This experiment was because BSA mimics the proteinaceous content of human body fluids. The results are shown in TABLES 11 and 12, below.

**TABLE 11 Effect of BSA added to Growth Media on % Surface Coverage by S. aureus**

| Surface | Rinsed % Surface Coverage by S. aureus | Std. Dev. | Post-Rinsed 14 days % Surface Coverage by S. aureus | Std. Dev. | Rinsing Difference | Rinsing Difference (%) |
|---|---|---|---|---|---|---|
| Ex.1 Flat (Compartive) | 72 | 15 | 84 | 15 | 12 | 117 |
| Ex. 10 Domes w/nano (Example) | 5 | 5 | 7 | 3 | 2 | 140 |

**TABLE 12 Effect of BSA added to Growth Media, Surface Coverage by P. aeruginosa**

| Surface | Rinsed % Surface Coverage by P. aeruginosa | Std. Dev. | Post-Rinsed 14 days % Surface Coverage by P. aeruginosa | Std. Dev. | Rinsing Difference | Rinsing Difference (%) |
|---|---|---|---|---|---|---|
| Ex.1 Flat (Compartive) | 80 | 13 | 88 | 17 | 8 | 110 |
| Ex. 10 Domes w/nano (Example) | 26 | 7 | 27 | 6 | 1 | 104 |

### Preconditioning with BSA

The Static Biofilm Assay described above was followed with the exception that after Step 4 an amount of 2 mL of a 3% BSA in phosphate buffered saline (PBS) was added to each well of the titer plate. The samples were left immersed in that BSA in PBS solution for 7 days, incubating at 37 degree C. The fluid level for each titer plate well was checked daily and additional BSA in PBS solution was added if necessary, enough to ensure the microstructure (and nanofeature) was covered (immersed) in the BSA in PBS solution. The results of the preconditioning with BSA experiment are shown in TABLE 13, below.

**TABLE 13. % Surface Coverage by E. coli after Preconditioning with BSA 7 days**

| Surface Name | % Surface Coverage by E. coli ATCC 700928 | Std. Dev. |
|---|---|---|
| Ex. 1 Flat (Compartive) | 91 | 11 * |
| Ex. 10 Domes w/nano (Example) | 19 | 7 |

### Preconditioning with FBS

The Static Biofilm Assay described above was followed with the exception that after Step 4 an amount of 2mL of Fetal Bovine Serum (FBS) sterile-filtered, suitable for cell culture, suitable for hybridoma, available from Sigma-Aldrich Chemical Company of Milwaukee, WI, was added to each well of the titer plate. The samples were left immersed in FBS for 7 days, incubating at 37 degree C. Fluid level for each titer plate well was checked daily and additional FBS was added if necessary, enough to ensure the microstructure (and nanofeature) was covered (immersed) in the FBS. The results of the preconditioning with FBS experiment are shown in TABLE 14, below.

**TABLE 14. % Surface Coverage by S. aureus after Preconditioning with FBS 7 days**

| Surface Name | % Surface Coverage by S. aureus ATCC 25923 | Std. Dev. |
|---|---|---|
| Ex. 1 Flat (Compartive) | 82 | 11 * |
| Ex. 7 (600 nm Rails w/nano) | 33 | 9 |
| Ex. 10 Domes w/nano (Example) | 9 | 3 |

### EMBODIMENTS

1. An apparatus having bacterial anti-adhesion properties comprising:
   a body having a engineered surface, at least a portion of said surface comprising a plurality of engineered structures;
   a plurality of randomly distributed, directed nanofeatures disposed on at least a portion of the engineered structures;
   wherein the plurality of engineered structures comprise at least one periodic structure, wherein the periodic structure comprises at least one dimension of at least 0.5 microns and no greater than 50 microns,
   wherein the pitch between adjacent structures of the plurality of engineered structures is at least 0.1 and no greater than 250 microns.
2. The apparatus of embodiment 1, wherein the plurality of engineered structures comprises a plurality of microstructures.
3. The apparatus of embodiment 1 or 2, wherein the nanofeatures comprise a majority thermoset polymer by weight.
4. The apparatus of embodiment 3, wherein the thermoset polymer is PDMS.
5. The apparatus of any of the previous embodiments, wherein the nanofeatures comprise an aspect ratio of at least 1 to 2 and no greater than 10 to 1.
6. The apparatus of embodiment 1, wherein the engineered structures comprises a plurality of engineered nanostructures.
7. The apparatus of embodiment 1, wherein the arrangement of the engineered structures on at least a portion of the engineered surface includes a plurality of unit cells, wherein each unit cell of the plurality of unit cells is at least partially defined by a dimension at least approximating the pitch and includes no more than one engineered structure; and wherein the plurality of unit cells are tiled.
8. The apparatus of embodiment 7, wherein the plurality of unit cells includes no more than three unique unit cell geometries.
9. The apparatus of embodiment 1, wherein the engineered structures are continuous structures.
10. The apparatus of embodiment 9, wherein the continuous structures comprises a rib or a rail.
11. The apparatus of any of the preceding embodiments, wherein the engineered structure is selected from the group consisting of a post, a pyramid, a rib, a diamond, a dome, and combinations thereof.
12. The apparatus of any of the previous embodiments, wherein the engineered surface comprises a substrate including a thermoplastic or thermoset material, and wherein each engineered structure comprises the same thermoplastic or thermoset material as the engineered surface.
13. The apparatus of embodiment 1 or 10, wherein each engineered structure of the plurality of engineered structures comprises an elastomeric structure.
14. The apparatus of embodiment 1, wherein the plurality of engineered structures protrude from the engineered surface.
15. The apparatus of embodiment 1, wherein the plurality of engineered structures are projected into the engineered surface.
16. The apparatus of embodiment 15, wherein the plurality of engineered structures comprise a plurality of discontinuous recesses.
17. The apparatus of embodiment 7, wherein the geometry of any unit cell of the plurality of unit cells is substantially identical to at least all neighboring unit cells.
18. The apparatus of any of the previous embodiments, wherein the colonization of *S*. *aureus* and *P. aeruginosa* on the portion of the engineered surface comprising the plurality of engineered structures and directed nanofeatures is significantly reduced according to the Static Biofilm Assay compared to the colonization on a flat surface comprised of the same material.
19. The apparatus of embodiment 1, wherein the engineered surface is on at least a portion of an orifice device.
20. The apparatus of embodiment 19, wherein the engineered surfaces is on at least a portion of a urinary catether, vascular access catheter, or endotracheal tube.
21. The apparatus of embodiment 19 wherein the engineered surfaces is on at least a portion of the interior of the orifice device.
22. The medical article of embodiment 19 wherein the engineered surfaces is on at least a portion of both the interior and the exterior of the orifice device.
23. The medical article of any one of embodiments 1-18, wherein the apparatus is a wound dressing, wound absorbent, or wound contact layer.
24. The medical article of any of the previous embodiments, wherein each engineered structure has a base and the largest cross-sectional dimension of the base is at least 1 micron and no greater than 2 microns.
25. The medical article of any of the previous embodiments, wherein at least a portion of the engineered surface comprises an antimicrobial.
26. A method of creating an anti-adhesion surface, the method comprising:
   providing a base device comprising an outer contact surface;
   creating a plurality of engineered structures on the outer contact surface, wherein the plurality of engineered structures comprise a pattern, said pattern comprising a periodic structure, and wherein the periodic structure comprises at least one dimension of at least 0.5 microns and no greater than 50 microns, and wherein the plurality of structures comprises a pitch of at least 0.5 microns and no greater than 150 microns;
   creating a plurality of directed nanofeatures on at least one of structure of the plurality of engineered structures.
27. The method of embodiment 26, wherein creating a plurality of engineered structures comprises creating a plurality of protrusions from the surface.
28. The method of embodiment 26, wherein creating the plurality of engineered structures comprises creating a plurality of discrete pockets in the contact surface.
29. The method of embodiment 27 or 28, wherein creating a plurality of engineered structures comprises replicating a plurality of microstructures on at least a portion of the contact surface.
30. The method of embodiment 26, wherein creating a plurality of microstructures comprises;
   replicating a plurality of microstructures on a polymeric sheath;
   securing the sheath to at least a portion of the contact surface.
31. The method of embodiment 30, wherein the polymeric sheath decreases in at least one dimension when exposed to heat, and wherein securing the sheath to at least a portion of the contact surface comprises applying the sheath to the contact surface and heating at least a portion of the sheath.
32. The method of any of the preceding embodiments, wherein the engineered structures comprise continuous structures.
33. The method of any of the preceding embodiments, wherein the at least one of the plurality of engineered structures is selected from the group consisting of a post, a pyramid, a diamond, a rib, a rail, a dome, and combinations thereof
34. The method of embodiment 26, wherein creating a plurality of nanofeatures comprises a vapor phase deposition.
35. The method of embodiment 26, wherein creating a plurality of nanofeatures comprises reactive ion-etching.
36. The method of anyone of embodiments, wherein creating a plurality of nanofeatures on at least one of engineered structure of the plurality of engineered structures comprises:
   applying a layer of nanoparticles to the engineered surface; and
   etching the surface using the layer of nanoparticles as an etch mask,
   resulting in a plurality of nanostructures on at least one of the microstructures.
37. The method of embodiment 36, wherein the nanoparticles are metal oxide nanoparticles.
38. The method of embodiment 37, wherein the metal oxide is ITO, CeO₂, ZrO₂, SiO₂, Al₂O₃ or TiO₂.
39. The method of embodiment 26, wherein creating the nanofeatures comprises lithographic printing a plurality of nanofeatures.
40. The method of anyone of embodiments 26-39, wherein the colonization of a target organism on the surface comprising the plurality of engineered structures and directed nanofeatures is at least 70% less than the colonization on a flat surface comprised of the same material when tested according to the Static Biofilm Assay.
41. A method of controlling microorganism adhesion to a medical article, the method comprising:
   providing a medical article having a surface comprising a plurality of engineered structures on at least a portion of the surface and a plurality of directed nanofeatures disposed on at least one of the structures, wherein each structure of the plurality of engineered structures comprises a base having at least one cross sectional microscale dimension, wherein the aspect ratio of each engineered structure is at least 0.5 and no greater than 10, wherein the pitch between adjacent structures of the plurality of engineered structures is at least 1 time and no greater than 5 times than the smallest cross- sectional dimension, wherein no base comprises a cross-sectional dimension greater than 20 microns; and
   placing the surface proximate a tissue or fluid, wherein the colonization of a target microorganism on the portion of the surface comprising the plurality of engineered microstructures is reduced in comparison to a flat surface comprised of the same material.
42. The method of embodiment 41, wherein the arrangement of the engineered structures on at least a portion of the device surface includes a plurality of unit cells, wherein each unit cell of the plurality of unit cells is at least partially defined by a dimension at least approximating the pitch, and wherein each unit cell comprises a boundary and each unit cell is directly adjacent the boundary of the nearest unit cell and wherein the plurality of unit cells includes no more than three unique unit cell geometries.

Various modifications and alterations to this invention will become apparent to those skilled in the art without departing from the scope of this invention as defined by the claims. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. An apparatus having bacterial anti-adhesion properties comprising:
a body having an engineered surface (110, 210, 700), at least a portion of said surface comprising a plurality of engineered structures (120, 220, 420, 710);
a plurality of randomly distributed, directed nanofeatures (140, 540, 550) disposed on at least a portion of the engineered structures (120, 220, 420, 710), wherein the nanofeatures (140, 540, 550) comprise inorganic nanoparticles and wherein the nanofeatures (140, 540, 550) comprise an aspect ratio of at least 1 to 2 and no greater than 10 to 1;
wherein the plurality of engineered structures (120, 220, 420, 710) comprise at least one periodic structure, wherein the periodic structure comprises at least one dimension (112, 114) of at least 0.5 microns and no greater than 50 microns, and
wherein the pitch between adjacent structures of the plurality of engineered structures (120, 220, 420, 710) is at least 0.1 and no greater than 150 microns.

2. The apparatus of claim 1, wherein the plurality of engineered structures (120, 220, 420, 710) comprises a plurality of microstructures.

3. The apparatus of claim 1 or 2, wherein the directed nanofeatures (140, 540, 550) comprise a majority of thermoset polymer by weight.

4. The apparatus of claim 1, wherein the engineered structures (120, 220, 420, 710) comprises a plurality of engineered nanostructures.

5. The apparatus of claim 1, wherein the arrangement of the engineered structures (120, 220, 420, 710) on at least a portion of the engineered surface (110, 210, 700) includes a plurality of unit cells (730), wherein each unit cell (740) of the plurality of unit cells (730) is at least partially defined by a dimension at least approximating the pitch and includes no more than one engineered structure (120, 220, 420, 710); and wherein the plurality of unit cells (730) are tiled.

6. The apparatus of claim 1, wherein the engineered structures (120, 220, 420, 710) are continuous structures.

7. The apparatus of any one of claims 1 to 5, wherein the engineered structure (120, 220, 420, 710) is selected from the group consisting of a post, a pyramid, a rib, a rail, a diamond, a dome, and combinations thereof.

8. The apparatus of claim 1, wherein the plurality of engineered structures (120, 220, 420, 710) protrude from the engineered surface (110, 210, 700).

9. The apparatus of claim 1, wherein the plurality of engineered structures (120, 220, 420, 710) are projected into the engineered surface (110, 210, 700).

10. The apparatus of claim 1, wherein the colonization of *S. aureus* and *P. aeruginosa* on the portion of the engineered surface (110, 210, 700) comprising the plurality of engineered structures (120, 220, 420, 710) and directed nanofeatures (140, 540, 550) is significantly reduced according to the Static Biofilm Assay compared to the colonization on a flat surface comprised of the same material.

11. The apparatus of claim 1, wherein the engineered surface (110, 210, 700) is on at least a portion of an orifice device.

12. The apparatus of claim 1, wherein the apparatus is a medical article selected from the group consisting of a wound dressing, a wound absorbent, and a wound contact layer.

13. The apparatus of claim 1, wherein at least a portion of the engineered surface (110, 210, 700) comprises an antimicrobial.

14. A method of creating an anti-adhesion surface, the method comprising:
providing a base device comprising an outer contact surface;
creating a plurality of engineered structures (120, 220, 420, 710) on the outer contact surface, wherein the plurality of engineered structures (120, 220, 420, 710) comprise a pattern, said pattern comprising a periodic structure, and wherein the periodic structure comprises at least one dimension (112, 114) of at least 0.5 microns and no greater than 50 microns, and wherein the plurality of structures (120, 220, 420, 710) comprises a pitch of at least 0.5 microns and no greater than 150 microns;
creating a plurality of directed nanofeatures (140, 540, 550) on at least one structure of the plurality of engineered structures (120, 220, 420, 710) by applying a layer of nanoparticles to the outer contact surface; and etching the surface using the layer of nanoparticles as an etch mask, wherein the nanofeatures (140, 540, 550) comprise an aspect ratio of at least 1 to 2 and no greater than 10 to 1.

15. The method of claim 14, wherein creating a plurality of engineered structures (120, 220, 420, 710) comprises;
replicating a plurality of microstructures on a polymeric sheath;
securing the sheath to at least a portion of the contact surface.

16. The method of any of claim 14 or 15, wherein the at least one of the plurality of engineered structures (120, 220, 420, 710) is selected from the group consisting of a post, a pyramid, a diamond, a rail, a rib, a dome, and combinations thereof.

17. The method of claim 16, wherein creating a plurality of nanofeatures (140, 540, 550) comprises reactive ion-etching.

18. The method of any one of claims 14 to 17, wherein the nanoparticles are metal oxide nanoparticles.

19. The apparatus of any one of claims 1 to 13 or the method of any one of claims 14 to 18, wherein the periodic structure comprises at least one cross-sectional dimension (112, 114) of at least 0.5 microns and no greater than 5 microns.

## Patentansprüche

1. Vorrichtung mit bakterienadhäsionshemmenden Eigenschaften, aufweisend:
einen Körper mit einer technisch hergestellten Oberfläche (110, 210, 700), wobei mindestens ein Teil der Oberfläche mehrere technisch hergestellte Strukturen (120, 220, 420, 710) aufweist;
mehrere zufällig verteilte, gerichtete Nanomerkmale (140, 540, 550), die mindestens an einem Teil der technisch hergestellten Strukturen (120, 220, 420, 710) angeordnet sind, wobei die Nanomerkmale (140, 540, 550) anorganische Nanoteilchen aufweisen und wobei die Nanomerkmale (140, 540, 550) ein Seitenverhältnis von mindestens 1 zu 2 und nicht größer als 10 zu 1 aufweisen;
wobei die mehreren technisch hergestellten Strukturen (120, 220, 420, 710) mindestens eine periodische Struktur aufweisen, wobei die periodische Struktur mindestens eine Abmessung (112, 114) von mindestens 0,5 Mikrometer und maximal 50 Mikrometer aufweist, und
wobei der Abstand zwischen benachbarten Strukturen der mehreren technisch hergestellten Strukturen (120, 220, 420, 710) mindestens 0,1 Mikrometer und maximal 150 Mikrometer beträgt.

2. Vorrichtung nach Anspruch 1, wobei die mehreren technisch hergestellten Strukturen (120, 220, 420, 710) mehrere Mikrostrukturen aufweisen.

3. Vorrichtung nach Anspruch 1 oder 2, wobei die gerichteten Nanomerkmale (140, 540, 550) nach Gewicht überwiegend Duroplast-Polymer aufweisen.

4. Vorrichtung nach Anspruch 1, wobei die technisch hergestellten Strukturen (120, 220, 420, 710) mehrere technisch hergestellte Nanostrukturen aufweisen.

5. Vorrichtung nach Anspruch 1, wobei die Anordnung der technisch hergestellten Strukturen (120, 220, 420, 710) an mindestens einem Teil der technisch hergestellten Oberfläche (110, 210, 700) mehrere Einheitszellen (730) aufweist, wobei jede Einheitszelle (740) der mehreren Einheitszellen (730) zumindest teilweise durch eine Abmessung definiert ist, die dem Abstand wenigstens näherungsweise entspricht, und maximal eine technische Struktur aufweist (120, 220, 420, 710); und wobei die mehreren Einheitszellen (730) kachelartig angeordnet sind.

6. Vorrichtung nach Anspruch 1, wobei die technisch hergestellten Strukturen (120, 220, 420, 710) durchgehende Strukturen sind.

7. Vorrichtung nach einem der Ansprüche 1 bis 5, wobei die technisch hergestellte Struktur (120, 220, 420, 710) aus der Gruppe ausgewählt ist, die aus einer Säule, einer Pyramide, einer Lamelle, einer Schiene, einem Diamanten, einer Kuppel und Kombinationen davon besteht.

8. Vorrichtung nach Anspruch 1, wobei die mehreren technisch hergestellten Strukturen (120, 220, 420, 710) von der technisch hergestellten Oberfläche (110, 210, 700) hervorstehen.

9. Vorrichtung nach Anspruch 1, wobei die mehreren technisch hergestellten Strukturen (120, 220, 420, 710) in die technisch hergestellte Oberfläche (110, 210, 700) hineinragen.

10. Vorrichtung nach Anspruch 1, wobei die Besiedelung von *S. aureus* und *P. aeruginosa* am Teil der technisch hergestellten Oberfläche (110, 210, 700), der die mehreren technisch hergestellten Strukturen (120, 220, 420, 710) und gerichteten Nanomerkmale (140, 540, 550) aufweist, gemäß der statischen Biofilm-Analyse im Vergleich zur Besiedlung auf einer flachen Oberfläche, die aus dem gleichen Material besteht, deutlich reduziert ist.

11. Vorrichtung nach Anspruch 1, wobei sich die technisch hergestellte Oberfläche (110, 210, 700) mindestens an einem Teil einer Öffnungsvorrichtung befindet.

12. Vorrichtung nach Anspruch 1, wobei die Vorrichtung ein Medizinprodukt ist, das aus der Gruppe bestehend aus einem Wundverband, einem Wund-Absorptionsmittel und einer Wundauflage ausgewählt ist.

13. Vorrichtung nach Anspruch 1, wobei mindestens ein Teil der technisch hergestellten Oberfläche (110, 210, 700) eine antimikrobielle Substanz aufweist.

14. Verfahren zur Bildung einer adhäsionshemmenden Oberfläche, wobei das Verfahren aufweist:
Bereitstellen einer Basisvorrichtung, die eine äußere Kontaktfläche aufweist;
Bilden mehrerer technisch hergestellter Strukturen (120, 220, 420, 710) an der äußeren Kontaktfläche, wobei die mehreren technisch hergestellten Strukturen (120, 220, 420, 710) ein Muster aufweisen, wobei das Muster eine periodische Struktur aufweist und wobei die periodische Struktur mindestens eine Abmessung (112, 114) von mindestens 0,5 Mikrometer und maximal 50 Mikrometer aufweist, und wobei die mehreren Strukturen (120, 220, 420, 710) einen Abstand von mindestens 0,5 Mikrometer und maximal 150 Mikrometer aufweisen;
Bilden mehrerer gerichteter Nanomerkmale (140, 540, 550) an mindestens einer Struktur der mehreren technisch hergestellten Strukturen (120, 220, 420, 710) durch Auftragen einer Schicht von Nanoteilchen auf der äußeren Kontaktfläche; und Ätzen der Oberfläche mit der Schicht Nanoteilchen als Ätzmaske, wobei die Nanomerkmale (140, 540, 550) ein Seitenverhältnis von mindestens 1 zu 2 und nicht größer als 10 zu 1 aufweisen.

15. Verfahren nach Anspruch 14, wobei das Bilden mehrerer technisch hergestellter Strukturen (120, 220, 420, 710) aufweist;
Replizieren mehrerer Mikrostrukturen auf einer Polymerhülle;
Befestigen der Hülle an mindestens einem Teil der Kontaktoberfläche.

16. Verfahren nach einem der Ansprüche 14 oder 15, wobei mindestens eine der mehreren technisch hergestellten Strukturen (120, 220, 420, 710) aus der Gruppe ausgewählt ist, die aus einer Säule, einer Pyramide, einem Diamanten, einer Schiene, einer Lamelle, einer Kuppel und Kombinationen daraus besteht.

17. Verfahren nach Anspruch 16, wobei das Bilden von mehreren Nanomerkmalen (140, 540, 550) reaktives lonenätzen aufweist.

18. Verfahren nach einem der Ansprüche 14 bis 17, wobei die Nanoteilchen Metalloxid-Nanoteilchen sind.

19. Vorrichtung nach einem der Ansprüche 1 bis 13 oder Verfahren nach einem der der Ansprüche 14 bis 18, wobei die periodische Struktur mindestens eine Querschnittsabmessung (112, 114) von mindestens 0,5 Mikrometer und maximal 5 Mikrometer aufweist.

## Revendications

1. Appareil possédant des propriétés anti-adhésives vis-à-vis des bactéries comprenant :
un corps présentant une surface produite techniquement (110, 210, 700), au moins une partie de ladite surface comprenant une pluralité de structures produites techniquement (120, 220, 420, 710) ;
une pluralité de nanoéléments orientés distribués de manière aléatoire (140, 540, 550) disposés sur au moins une partie des structures produites techniquement (120, 220, 420, 710), dans lequel les nanoéléments (140, 540, 550) comprennent des nanoparticules inorganiques et dans lequel les nanoéléments (140, 540, 550) comprennent un rapport d'aspect égal ou supérieur à 1 sur 2 et inférieur ou égal à 10 sur 1 ;
dans lequel la pluralité de structures produites techniquement (120, 220, 420, 710) comprend au moins une structure périodique, dans lequel la structure périodique comprend au moins une dimension (112, 114) égale ou supérieure à 0,5 micron et inférieure ou égale à 50 microns, et
dans lequel le pas entre les structures adjacentes de la pluralité de structures produites techniquement (120, 220, 420, 710) est égal ou supérieur à 0,1 et inférieur ou égal à 150 microns.

2. Appareil selon la revendication 1, dans lequel la pluralité de structures produites techniquement (120, 220, 420, 710) comprend une pluralité de microstructures.

3. Appareil selon la revendication 1 ou 2, dans lequel les nanoéléments orientés (140, 540, 550) comprennent une majorité de polymères thermodurcissables en poids.

4. Appareil selon la revendication 1, dans lequel les structures produites techniquement (120, 220, 420, 710) comprennent une pluralité de nanostructures produites techniquement.

5. Appareil selon la revendication 1, dans lequel l'agencement des structures produites techniquement (120, 220, 420, 710) sur au moins une partie de la surface produite techniquement (110, 210, 700) inclut une pluralité de cellules unitaires (730), dans lequel chaque cellule unitaire (740) de la pluralité de cellules unitaires (730) est au moins partiellement définie par une dimension au moins approximativement égale au pas et n'inclut pas plus d'une structure produite techniquement (120, 220, 420, 710) ; et dans lequel la pluralité de cellules unitaires (730) est disposée en mosaïque.

6. Appareil selon la revendication 1, dans lequel les structures produites techniquement (120, 220, 420, 710) sont des structures continues.

7. Appareil selon l'une quelconque des revendications 1 à 5, dans lequel la structure produite techniquement (120, 220, 420, 710) est choisie dans le groupe constitué des éléments suivants : un poteau, une pyramide, une nervure, un rail, un diamant, un dôme et des combinaisons de ceux-ci.

8. Appareil selon la revendication 1, dans lequel la pluralité de structures produites techniquement (120, 220, 420, 710) fait saillie depuis la surface produite techniquement (110, 210, 700).

9. Appareil selon la revendication 1, dans lequel les structures de la pluralité de structures produites techniquement (120, 220, 420, 710) font saillie dans la surface produite techniquement (110, 210, 700).

10. Appareil selon la revendication 1, dans lequel la colonisation de *S. aureus* et *P. aeruginosa* sur la partie de la surface produite techniquement (110, 210, 700) comprenant la pluralité de structures produites techniquement (120, 220, 420, 710) et de nanoéléments orientés (140, 540, 550) est considérablement réduite selon le test Static Biofilm Assay par rapport à la colonisation sur une surface plane constituée du même matériau.

11. Appareil selon la revendication 1, dans lequel la surface produite techniquement (110, 210, 700) se trouve sur au moins une partie d'un dispositif d'orifice.

12. Appareil selon la revendication 1, dans lequel l'appareil est un article médical choisi dans le groupe constitué des éléments suivants : un pansement pour plaie, un produit absorbant pour plaie et une couche de contact avec une plaie.

13. Appareil selon la revendication 1, dans lequel au moins une partie de la surface produite techniquement (110, 210, 700) comprend un agent antimicrobien.

14. Procédé de création d'une surface anti-adhésive, le procédé comprenant les étapes consistant à :
fournir un dispositif de base comprenant une surface de contact extérieure ;
créer une pluralité de structures produites techniquement (120, 220, 420, 710) sur la surface de contact extérieure, dans lequel la pluralité de structures produites techniquement (120, 220, 420, 710) comprend un motif, ledit motif comprenant une structure périodique, et dans lequel la structure périodique comprend au moins une dimension (112, 114) d'au moins 0,5 micron et d'au plus 50 microns, et dans lequel la pluralité de structures (120, 220, 420, 710) comprend un pas d'au moins 0,5 micron et d'au plus 150 microns ;
créer une pluralité de nanoéléments orientés (140, 540, 550) sur au moins une structure de la pluralité de structures produites techniquement (120, 220, 420, 710) en appliquant une couche de nanoparticules sur la surface de contact extérieure ; et graver la surface en utilisant la couche de nanoparticules comme masque de gravure, dans lequel les nanoéléments (140, 540, 550) comprennent un rapport d'aspect égal ou supérieur à 1 sur 2 et inférieur ou égal à 10 sur 1.

15. Procédé selon la revendication 14, dans lequel la création d'une pluralité de structures produites techniquement (120, 220, 420, 710) comprend les étapes consistant à :
répliquer une pluralité de microstructures sur une gaine polymère ;
fixer la gaine sur au moins une partie de la surface de contact.

16. Procédé selon l'une quelconque des revendications 14 ou 15, dans lequel l'une au moins parmi la pluralité de structures produites techniquement (120, 220, 420, 710) est choisie dans le groupe constitué des éléments suivants : un poteau, une pyramide, un diamant, un rail, une nervure, un dôme et des combinaisons de ceux-ci.

17. Procédé selon la revendication 16, dans lequel la création d'une pluralité de nanoéléments (140, 540, 550) comprend une gravure ionique réactive.

18. Procédé selon l'une quelconque des revendications 14 à 17, dans lequel les nanoparticules sont des nanoparticules d'oxyde métallique.

19. Appareil selon l'une quelconque des revendications 1 à 13, ou procédé selon l'une quelconque des revendications 14 à 18, dans lequel la structure - périodique comprend au moins une dimension en coupe transversale (112, 114) égale ou supérieure à 0,5 micron et inférieure ou égale à 5 microns.
